(19) Europäisches Patentamt European Patent Office Office européen des brevets

(11) **EP 3 883 591 B1**

(12) **EUROPEAN PATENT SPECIFICATION**

(45) Date of publication and mention of the grant of the patent:
**13.11.2024 Bulletin 2024/46**

(21) Application number: **19816211.7**

(22) Date of filing: **20.11.2019**

(51) International Patent Classification (IPC):
*A61K 38/17* (2006.01)    *A61K 38/22* (2006.01)
*A61P 9/10* (2006.01)    *C12N 15/861* (2006.01)

(52) Cooperative Patent Classification (CPC):
(C-Sets available)
**A61K 38/1796; A61K 38/2221; A61K 48/005;**
**A61P 9/10; C12N 15/86;** A01K 2207/15;
A01K 2217/052; A01K 2227/105; A01K 2267/0375;
C12N 2750/14143    (Cont.)

(86) International application number:
**PCT/EP2019/081962**

(87) International publication number:
**WO 2020/104540 (28.05.2020 Gazette 2020/22)**

(54) **RELAXIN RECEPTOR 1 FOR USE IN TREATMENT AND PREVENTION OF HEART FAILURE**

RELAXINREZEPTOR 1 ZUR VERWENDUNG IN DER BEHANDLUNG UND VORBEUGUNG VON HERZVERSAGEN

RÉCEPTEUR 1 DE RELAXINE DESTINÉ À ÊTRE UTILISÉ DANS LE TRAITEMENT ET LA PRÉVENTION DE L'INSUFFISANCE CARDIAQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **20.11.2018 EP 18207296**

(43) Date of publication of application:
**29.09.2021 Bulletin 2021/39**

(73) Proprietor: **Universität Heidelberg**
**69117 Heidelberg (DE)**

(72) Inventors:
• **KATUS, Hugo**
**69120 Heidelberg (DE)**
• **RAAKE, Philip Wolfram Joseph**
**69214 Eppelheim (DE)**
• **SCHLEGEL, Philipp**
**69115 Heidelberg (DE)**
• **SASIPONG, Nuttarak**
**69115 Heidelberg (DE)**

(74) Representative: **Altmann Stößel Dick**
**Patentanwälte PartG mbB**
**Theodor-Heuss-Anlage 2**
**68165 Mannheim (DE)**

(56) References cited:
**EP-A1- 2 946 788    WO-A2-2005/052123**

• **NITIN A PATIL ET AL: "Relaxin family peptides: structure-activity relationship studies", BRITISH JOURNAL OF PHARMACOLOGY, WILEY-BLACKWELL, UK, vol. 174, no. 10, 19 January 2017 (2017-01-19), pages 950 - 961, XP071171594, ISSN: 0007-1188, DOI: 10.1111/BPH.13684**
• **HALLS M L ET AL: "Relaxin Family Peptide Receptors - former orphans reunite with their parent ligands to activate multiple signalling pathways", BRITISH JOURNAL OF PHARMACOLOGY, WILEY-BLACKWELL, UK, vol. 150, no. 6, 29 January 2009 (2009-01-29), pages 677 - 691, XP071123030, ISSN: 0007-1188, DOI: 10.1038/SJ.BJP.0707140**

- SETHI ASHISH ET AL: "The complex binding mode of the peptide hormone H2 relaxin to its receptor RXFP1", NATURE COMMUNICATIONS, vol. 7, no. 1, 18 April 2016 (2016-04-18), XP093018329, Retrieved from the Internet <URL:https://www.nature.com/articles/ncomms11344> DOI: 10.1038/ncomms11344
- ANONYMOUS: "RXFP1 | Relaxin family peptide receptors | IUPHAR/BPS Guide to PHARMACOLOGY", 8 August 2017 (2017-08-08), XP093018303, Retrieved from the Internet <URL:https://web.archive.org/web/20170808165240/https://www.guidetopharmacology.org/GRAC/ObjectDisplayForward?objectId=351> [retrieved on 20230127]
- ANONYMOUS: "5. Symposium des Cardiology Career Program 2015", 21 March 2015 (2015-03-21), XP055661910, Retrieved from the Internet <URL:https://www.klinikum.uni-heidelberg.de/fileadmin/medizinische_klinik/Abteilung_3/ARCHIV/2015/150216MED-3_FL_DM_Symp-CCP_ID21676.pdf> [retrieved on 20200127]
- ANONYMOUS: "Abstract 870: Cardiac Gene Therapy With Relaxin Receptor 1 Overexpression Protects Against Acute Myocardial Infarction and Associated Adverse Remodeling | Circulation Research", 16 October 2019 (2019-10-16), XP055661893, Retrieved from the Internet <URL:https://www.ahajournals.org/doi/abs/10.1161/res.125.suppl_1.870> [retrieved on 20200127]
- ANONYMOUS: "Targeted Gene Therapy with RXFP1 Attenuates Myocardial Infarction and Preserves Left Ventricular Function in Mice | The FASEB Journal", 20 April 2018 (2018-04-20), XP055661897, Retrieved from the Internet <URL:https://www.fasebj.org/doi/abs/10.1096/fasebj.2018.32.1_supplement.580.14> [retrieved on 20200127]
- HSU SHEAU YU ET AL: "Activation of orphan receptors by the hormone relaxin", SCIENCE, AMERICAN ASSOCIATION FOR THE ADVANCEMENT OF SCIENCE, vol. 295, no. 5555, 1 January 2002 (2002-01-01), pages 671 - 674, XP002196451, ISSN: 0036-8075, DOI: 10.1126/SCIENCE.1065654
- TEERLINK J R ET AL: "Relaxin for the treatment of patients with acute heart failure (Pre-RELAX-AHF): a multicentre, randomised, placebo-controlled, parallel-group, dose-finding phase IIb study", LANCET, ELSEVIER, AMSTERDAM, NL, vol. 373, no. 9673, 25 April 2009 (2009-04-25), pages 1429 - 1439, XP026023566, ISSN: 0140-6736, [retrieved on 20090423], DOI: 10.1016/S0140-6736(09)60622-X

Remarks:
The complete document including Reference Table(s) and the Sequence Listing(s) can be downloaded from the EPO website

(52) Cooperative Patent Classification (CPC): (Cont.)

C-Sets
A61K 38/1796, A61K 2300/00;
A61K 38/2221, A61K 2300/00

**Description**

The present invention relates to a polynucleotide comprising an expressible nucleic acid sequence encoding a relaxin family peptide receptor (RXFP) polypeptide for use in treatment and/or prevention of heart failure in a subject according to claim 1. The present invention further relates to a vector comprising the polynucleotide of the present invention for use in treatment and/or prevention of heart failure according to claim 11, as well as to host cells for use according to claim 12, and to RXFP1 agonists for use according to claim 13.

Treatment options for heart failure at present focus on alleviating symptoms and preventing progression of disease, while causative treatment usually is restricted to addressing underlying risk factors like infections, alcohol ingestion, anemia, and others. Thus, treatments may be pharmacological treatment (i.e. with Beta blockers, etc.), non-surgical device treatment (i.e. cardiac resynchronization therapy), or surgical treatment (i.e. left ventricular assist device, total artificial heart implantation, or heart transplantation). However, not all treatment options are open for all patients, e.g. heart transplantation may not be available for older patients due to contraindications stated by transplant guidelines.

More recently, gene therapeutic approaches were tested for treatment of heart failure (Greenberg et al. (2016), Lancet 387:1178); as vectors, adeno-associated virus (AAV) vectors are frequently used, with different genotypes having different efficacy in transferring genetic material to the heart (Bish et al. (2008), Human Gene Therapy 19:1359; Naso et al. (2017), BioDrugs 31:317). Moreover, chimeric and mutant AAV strains were used (Asokan (2012, Mol Ther 20(4):699). In one such approach, it was found that targeted gene therapy with RXFP1 attenuates myocardial infarction and preserves left ventricular function in mice (Devarakonda et al., FASEB J, doi.org/10.1096/fasebj.2018.32.1_supplement.580.14)

The peptide hormone relaxin was found to be upregulated in heart failure patients and to have a positive correlation to severity of disease. However, administration of a recombinant relaxin derivative, while showing subjective improvement of dyspnea, did not provide for significant effects on the secondary endpoints of cardiovascular death or readmission to hospital for heart failure or renal failure in a clinical trial (Teerlink et al. (2013), Lancet 381:29).

There is, thus, a need in the art to provide improved means and methods for treatment of heart failure. In particular, there is a need to provide means and methods avoiding at least in part the drawbacks of the prior art as discussed above.

This problem is solved by the subject matter of the present invention with the features of the independent claims. Preferred embodiments, which might be realized in an isolated fashion or in any arbitrary combination are listed in the dependent claims.

Accordingly, the present invention relates to a polynucleotide comprising an expressible nucleic acid sequence encoding a relaxin family peptide receptor (RXFP) polypeptide for use according to claim 1; further, generic disclosure relates to a polynucleotide comprising an expressible nucleic acid sequence encoding a relaxin family peptide receptor (RXFP) polypeptide for use in treatment and/or prevention of heart failure in a subject.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%. Further, the term "essentially" indicates that deviations having influence on the indicated result or use are absent, i.e. potential deviations do not cause the indicated result to deviate by more than $\pm$ 20%, more preferably $\pm$ 10%, most preferably $\pm$ 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "relaxin", as used herein, relates to the family of peptide hormones known to the skilled person under this designation. In a preferred embodiment, the relaxin is a relaxin-1 or relaxin-3, more preferably a mammalian relaxin-1 or relaxin-3, preferably including recombinant variants and isoforms thereof. Preferably, the relaxin is a relaxin-1, more preferably a mammalian relaxin-1, preferably including recombinant variants and isoforms thereof. More preferably, the relaxin is a human, rat, mouse, or pig relaxin-1, even more preferably an isoform of human relaxin-1, preferably having an amino acid sequence at least 70% identical to the amino acid sequence of SEQ ID NO:9; more preferably comprising, most preferably consisting of, the amino acid sequence of SEQ ID NO:9. Also preferably, relaxin is a relaxin-2, more preferably a mammalian relaxin-2, preferably including recombinant variants and isoforms thereof. More preferably the relaxin is a human, rat, mouse, or pig relaxin-2, more preferably a recombinant variant or an isoform of human relaxin-2.

Preferably, the relaxin-2 consists of a heavy and a light chain, wherein the heavy chain comprises, preferably consists of, an amino acid sequence at least 70% identical to the amino acid sequence of SEQ ID NO:10; more preferably comprises, most preferably consists of, the amino acid sequence of SEQ ID NO:10; and wherein the light chain comprises, preferably consists of, an amino acid sequence at least 70% identical to the amino acid sequence of SEQ ID NO:11; more preferably comprises, most preferably consists of, the amino acid sequence of SEQ ID NO:11. Preferably, said relaxin-2 comprises covalent disulfide bonds between amino acid 11 of the heavy chain and amino acid 11 of the light chain, between amino acid 23 of the heavy chain and amino acid 24 of the light chain, and/or between amino acids 10 and 15 of the light chain. Thus, preferably, the relaxin is serelaxin. The minimal active structure of relaxin-2 has been identified (Hossain et al. (2011), JBC 286(43):37555); thus, preferably, the relaxin-2 consists of a heavy and a light chain, wherein the heavy chain comprises, preferably consists of, an amino acid sequence at least 70% identical to the amino acid sequence of SEQ ID NO:12; more preferably comprises, most preferably consists of, the amino acid sequence of SEQ ID NO:12; and wherein the light chain comprises, preferably consists of, an amino acid sequence at least 70% identical to the amino acid sequence of SEQ ID NO:13; more preferably comprises, most preferably consists of, the amino acid sequence of SEQ ID NO:13. In a preferred embodiment, the relaxin is human relaxin-3, Genbank Acc No. EAW84388.1, SEQ ID NO:38.

The term "relaxin family peptide receptor", or "RXFP", relates to any member of the family of relaxin family peptide receptors, also known to the skilled person as relaxin receptors, having the biological activity of mediating G-protein coupled signaling from relaxin peptide hormones. The RXFP is an RXFP1. Preferably, the RXFP has an amino acid sequence at least 70% identical to SEQ ID NO: 4. More preferably, the RXFP is a mammalian homolog of human RXFP1, still more preferably is a recombinant variant or an isoform of a human, rat, mouse, or pig RXFP1; even more preferably, the RXFP is one of the isoforms of human RXFP1, preferably isoform 1 (SEQ ID NO:2; Genbank Acc No.:NP_067647.2) or isoform 2 (SEQ ID NO:4; Genbank Acc No.:NP_001240656.1). The term "RXFP polypeptide", as used herein, preferably includes polypeptide variants of the aforesaid RXFP polypeptides.

As used herein, the term "polypeptide variant" relates to any chemical molecule comprising at least one polypeptide or fusion polypeptide as specified elsewhere herein, having the indicated activity, but differing in primary structure from said polypeptide or fusion polypeptide indicated. Thus, the polypeptide variant, preferably, is a mutein having the indicated activity. Preferably, the polypeptide variant comprises a peptide having an amino acid sequence corresponding to an amino acid sequence of 50% to 100%, more preferably 70 to 95%, even more preferably 80% to 90% of consecutive amino acids comprised in a polypeptide as specified herein. Moreover, also encompassed are further polypeptide variants of the aforementioned polypeptides. Such polypeptide variants have at least essentially the same biological activity as the specific polypeptides. Moreover, it is to be understood that a polypeptide variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino acid sequence of the specific polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Polypeptide variants referred to herein may be allelic variants or any other species specific homologs, paralogs, or orthologs. Moreover, the polypeptide variants referred to herein include fragments of the specific polypeptides or the aforementioned types of polypeptide variants as long as these fragments and/or variants have the biological activity as referred to above. Such fragments may be or be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics.

Preferably, the RXFP polypeptide variant is a chimeric RXFP polypeptide. The term "chimeric" is understood by the skilled person to relate to any molecule composed of components originating from at least two different species or strains. Thus, preferably, the RXFP polypeptide variant comprises amino acid sequences derived from RXFP polypeptides of at least two subject species as specified elsewhere herein. Thus, preferably, the extracellular receptor portion of the RXFP polypeptide may e.g. be a mouse extracellular RXFP receptor portion, whereas the transmembrane and/or intracellular portions may be human RXFP transmembrane and/or intracellular portions, e.g. to provide an RXFP polypeptide providing relaxin signaling in human cells using mouse relaxin as an agonist; or the extracellular receptor portion of the RXFP polypeptide may e.g. be a human extracellular RXFP receptor portion, whereas the transmembrane and/or intracellular portions may be mouse RXFP transmembrane and/or intracellular portions, e.g. to provide an RXFP polypeptide providing relaxin signaling in human cells using a mouse relaxin intracellular domain.

The term "polynucleotide", as used herein, relates to a polynucleotide comprising a nucleic acid sequence which encodes a relaxin family peptide receptor (RXFP) polypeptide as specified herein above, the RXFP polypeptide having the biological activity as described above. Preferably, the polynucleotide comprises, more preferably consists of, the nucleic acid sequence of SEQ ID NO:1, 3, or 5. It is to be understood that a polypeptide having an amino acid sequence as detailed above may also be encoded, due to the degenerated genetic code, by more than one species of polynucleotide. Thus, preferably, the polynucleotide encodes a polypeptide comprising, preferably consisting of, an amino acid sequence at least 70% identical to the amino acid sequence of SEQ ID NO:2 or/and 4. More preferably, the polynucleotide encodes a polypeptide comprising, preferably consisting of, the amino acid sequence of SEQ ID NO:2 or/and 4. Preferably, the polynucleotide comprises, preferably consists of, a nucleic acid sequence at least 70% identical to the nucleic acid sequence of SEQ ID NO:3. More preferably, the polynucleotide comprises, preferably consists of, the amino acid sequence of SEQ ID NO:3.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still encode an RXFP polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in standard text books. A preferred example for stringent hybridization conditions are hybridization conditions in 6x sodium chloride/sodium citrate (= SSC) at approximately 45°C, followed by one or more wash steps in 0.2x SSC, 0.1% SDS at 50°C to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks. Alternatively, polynucleotide variants are obtainable by PCR-based techniques such as mixed oligonucleotide primer-based amplification of DNA, i.e. using degenerated primers against conserved domains of the polypeptides of the present invention. Conserved domains of the polypeptides of the present invention may be identified by a sequence comparison of the nucleic acid sequence of the polynucleotide or of the amino acid sequence of the polypeptides as specified above. Suitable PCR conditions are well known in the art. As a template, DNA or cDNA from mammalian cells may be used. Further, variants include polynucleotides comprising nucleic acid sequences which are at least 70%, at least 75%, at least 80%, at least 85%, at least 90%, at least 95%, at least 98% or at least 99% identical to the nucleic acid sequences detailed above. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences referred to above. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981)), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. A polynucleotide comprising a fragment of any of the aforementioned nucleic acid sequences is also encompassed as a polynucleotide. The fragment shall encode a polypeptide which still has the biological activity as specified above. Accordingly, the polypeptide may comprise or consist of the domains of the polypeptide of the present invention conferring the said biological activity. A fragment as meant herein, preferably, comprises at least 500, at least 1000, at least 1500 or at least 2000 consecutive nucleotides of any one of the aforementioned nucleic acid sequences or encodes an amino acid sequence comprising at least 200, at least 300, at least 500, or at least 700 consecutive amino acids of any one of the aforementioned amino acid sequences. The polynucleotides either essentially consist of the aforementioned nucleic acid sequences or comprise the aforementioned nucleic acid sequences. Thus, they may contain further nucleic acid sequences as well. Specifically, the polynucleotides of the present invention may encode fusion proteins wherein one partner of the fusion protein is a polypeptide being encoded by a nucleic acid sequence recited above. Such fusion proteins may, preferably, comprise as additional part polypeptides for monitoring expression (e.g., green, yellow, blue or red fluorescent proteins, alkaline phosphatase and the like) or one or more of so-called "tags" which may serve as a detectable marker or as an auxiliary measure for purification purposes. Tags for the different purposes are well known in the art and comprise FLAG-tags, 6-histidine-tags, MYC-tags and the like. The polynucleotide shall be provided, preferably, either as an isolated polynucleotide (i.e. isolated from its natural context) or in genetically modified form. The polynucleotide, preferably, is DNA, including cDNA, or RNA. The term encompasses single- as well as double-stranded polynucleotides. Moreover, preferably comprised are also chemically modified polynucleotides including naturally occurring modified polynucleotides such as glycosylated or methylated polynucleotides or artificially modified ones such as biotinylated polynucleotides.

The term "expressible nucleic acid sequence", as used herein, relates to a nucleic acid sequence of a polynucleotide operatively linked to at least one expression control sequence causing transcription of the nucleic acid sequence comprised in said polynucleotide to occur, preferably in eukaryotic cells or isolated fractions thereof, preferably into a translatable mRNA. Regulatory elements ensuring expression in eukaryotic cells, preferably mammalian cells, are well known in the art. They, preferably, comprise regulatory sequences ensuring initiation of transcription, in particular at least one promoter, and, optionally, poly-A signals ensuring termination of transcription and stabilization of the transcript. Preferably, regulatory are cell- or tissue-type specific and/or are inducible, e.g. by administration of specific inducers. Preferred constitutive promoters are e.g. the CMV-, SV40-, or RSV (Rous sarcoma virus)-promoter, CMV-enhancer, or SV40-enhancer. Preferably, the promoter is a heart-cell specific promoter, even more preferably a cardiomyocyte-specific promoter, most preferably an MLC260 promoter. Preferably, said MLC260 promoter comprises, preferably consists of, a nucleic acid sequence at least 70% identical to the nucleic acid sequence of SEQ ID NO:7; more preferably, the MLC260 promoter comprises, preferably consists of, the nucleic acid sequence of SEQ ID NO:7. Additional regulatory elements may include transcriptional as well as translational enhancers. Preferably, the enhancer is a CMV enhancer, more preferably comprising, preferably consisting of, a nucleic acid sequence at least 70% identical to the nucleic acid sequence of SEQ ID NO:6; more preferably, comprising, preferably consisting of, the nucleic acid sequence of SEQ ID NO:6.

Preferably, the polynucleotide further comprises at least one packaging signal for a vector. The term "vector", preferably, encompasses phage, plasmid, viral or retroviral vectors as well as artificial chromosomes, such as bacterial or yeast artificial chromosomes. More preferably, vector relates to a vector derived from an AAV. Moreover, the term also relates to targeting constructs which allow for random or site-directed integration of the targeting construct into genomic DNA. Such targeting constructs, preferably, comprise DNA of sufficient length for either homologous or heterologous recombination. The vector encompassing the polynucleotides as specified herein, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Viral vectors may be replication competent or replication defective. In the latter case, viral propagation generally will occur only in complementing hosts and/or cells. More preferably, in the vector, the polynucleotide is operatively linked to expression control sequences as specified herein above. Preferred expression vectors are vectors derived from viruses such as retroviruses, in particular lentiviruses, vaccinia virus, adeno-associated virus, herpes viruses, or bovine papilloma virus, which may be used for delivery of the polynucleotides or vector of the invention into targeted cell population. Methods used to construct recombinant viral vectors are well known to those skilled from standard text books. Preferably, the vector is an AAV vector, more preferably a self-complementary AAV vector, as described in e.g. Grimm et al. (2006), J Virol 80:426. Preferably, the vector is an adeno-associated virus (AAV), more preferably is an AAV1, AAV5, AAV6, or an AAV9. More preferably, the AAV is an AAV9 or AAV6, still more preferably an AAV9. Thus, preferably, the polynucleotide is comprised in a vector packaging construct, preferably a viral vector packaging construct, more preferably an adeno-associated virus (AAV) packaging construct, preferably an AAV9 or AAV6 packaging construct, more preferably an AAV9 packaging construct. Also preferably, the polynucleotide is comprised in a vector, preferably a viral vector, more preferably an adeno-associated virus (AAV) vector, most preferably an AAV9 or AAV6 vector, still more preferably an AAV9 vector. In a preferred embodiment, the vector is an AAV1, AAV2, AAV5, AAV6, or an AAV9

As used herein, the term "Adeno-Associated Virus" or "AAV" relates to the group of viruses containing a short (approx. 4.7 kB) single-stranded DNA and depending in their lytic replication on the presence of an Adenovirus. AAVs are members of the Parvoviridae family of viruses. Thus, AAV vectors, as used herein, are vectors derived from AAV, i.e. gene transfer vehicles using the capsid polypeptide of AAV to mediate the transfer of recombinant polynucleotides into target cells. The term "AAV capsid polypeptide" as referred to herein relates to a polypeptide with the activity of self-assembly to produce the proteinaceous shell of an AAV particle, also referred to as coat protein or VP protein. It is to be understood that not all AAV capsid polypeptide molecules in a given cell assemble into AAV capsids. Preferably, at least 25%, at least 50%, at least 75%, at least 85%, at least 90%, at least 95% of all AAV capsid polypeptide molecules assemble into AAV capsids. Suitable assays for measuring this biological activity are described e.g. in Smith-Arica and Bartlett (2001), Curr Cardiol Rep 3(1): 43. Preferably, the AAV capsid polypeptide is the capsid polypeptide of AAV9 (Genbank Acc. No: AAS99264.1), AAV1 (Genbank Acc. No: AAD27757.1), AAV2 (Genbank Acc. No: AAC03780.1), AAV3 (Genbank Acc. No: AAC55049.1), AAV3b (Genbank Acc. No: AF028705.1), AAV4 (Genbank Acc. No: AAC58045.1), AAV5 (Genbank Acc. No: AAD13756.1), AAV6 (Genbank Acc. No: AF028704.1), AAV7 (Genbank Acc. No: AAN03855.1), AAV8 (Genbank Acc. No: AAN03857.1), AAV10 (Genbank Acc. No: AAT46337.1), AAVrh10 (Genbank Acc. No: AY243015.1), AAV11 (Genbank Acc. No: AAT46339.1), AAV12 (Genbank Acc. No: ABI16639.1), or AAV13 (Genbank Acc. No: ABZ10812.1), AAVpo1 (Genbank Acc. No: FJ688147.1). More preferably, the AAV capsid polypeptide is the capsid polypeptide of AAV9 (Genbank Acc. No: AAS99264.1).

As used herein, the term AAV capsid polypeptide preferably includes AAV capsid polypeptide variants, the term "polypeptide variants" being used as specified herein above, said polypeptide variants having the aforesaid activity. Preferably, the AAV capsid polypeptide variant is a chimeric capsid polypeptide. The term "chimeric", as specified herein above, is understood by the skilled person to relate to any molecule composed of components originating from at least two different species or strains. Thus, preferably, the capsid polypeptide variant comprises amino acid sequences derived from capsid polypeptides of at least two, preferably at least three AAV strains. More preferably, the capsid polypeptide variant comprises amino acid sequences of at least five, preferably at least ten, more preferably at least 20 contiguous amino acids derived from capsid polypeptides of at least two, preferably at least three AAV strains. Preferably, the polynucleotide encoding said chimeric capsid polypeptide was generated by DNA shuffling, a technique which is, in principle, known to the skilled person. Also preferably, the AAV capsid polypeptide variant comprises a binding peptide inserted into the AAV capsid polypeptide amino acid sequence, wherein preferably the insertion site of the binding peptide corresponds to amino acid 588 or 589, preferably 588, of the AAV9 capsid polypeptide. Whether an insertion site in an AAV capsid polypeptide corresponds to an insertion site in the AAV9 capsid polypeptide can be established by the skilled person by known methods, preferably by aligning the amino acid sequences of the capsid polypeptides. The term "binding peptide", as used herein, relates to a short peptide, preferably of a length of from 2 to 15 amino acids, increasing affinity of an AAV capsid polypeptide to a host cell of interest. Appropriate binding peptides are, in principle, known in the art.

The term "treatment" refers to an amelioration of a disease or disorder referred to herein or the symptoms accompanied therewith to a significant extent. Said treating as used herein also includes an entire restoration of health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99%. The p-values are, preferably, 0.1, 0.05, 0.01, 0.005, or 0.0001. Preferably, the treatment shall be effective for at least 20%, more preferably at least 50%, even more preferably at least 60%, still more preferably at least 70%, still more preferably at least 80%, most preferably at least 90% of the subjects of a given cohort or population. As will be understood by the skilled person, effectiveness of treatment is dependent on a variety of factors including, e.g. disease stage and severity. As will be understood, the term treatment includes measures preventing and/or delaying progression of a disease or disorder referred to herein.

The tern "preventing" refers to retaining health with respect to the diseases or disorders referred to herein for a certain period of time in a subject. It will be understood that said period of time is dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification. As used herein, the term prevention, preferably, includes measures of gene therapy, as described elsewhere herein. Thus, the subject receiving preventive measures may be free of any symptoms of disease, but may be identified e.g. by gene analysis to have an increased risk of developing a disease or disorder referred to herein, or the subject receiving preventive measures may be identified by cardiac strain analysis via echocardiogram or MRI.

The term "heart failure" is known to the skilled person to relate to a pathological condition in which the heart of a subject is unable to maintain sufficient blood flow to meet the subject's body's needs. Preferably, heart failure is chronic heart failure or acute deterioration of pre-existing heart failure. Also preferably, heart failure is heart failure with preserved ejection fraction, heart failure with mid-range ejection fraction, or heart failure with reduced ejection fraction. More preferably, heart failure is systolic heart failure or diastolic heart failure, preferably with mid-range ejection fraction or with reduced ejection fraction. Most preferably, heart failure is systolic heart failure, preferably with reduced ejection fraction.

The term "subject", as used herein, relates to a vertebrate, more preferably a mammal, even more preferably a livestock or companion animal, such as a chicken, a goose, a duck, a goat, a sheep, a cattle, a pig, a horse, a dog, a cat, a hamster, a rat, a mouse, an alpaca, a guinea pig, a rabbit, a hare, or a human. Most preferably, the subject is a human. Preferably, the subject is suffering, preferably at the time of administration of a treatment as specified herein, suffering from heart failure, more preferably was diagnosed to suffer from heart failure. Symptoms and diagnostic methods for diagnosing heart failure are known to the skilled person and include in particular fatigue, shortness of breath and other well-known symptoms; diagnostic tests for heart failure include in particular clinical chemistry, genetic testing, electrocardiogram, echocardiography (transthoracic and transesophageal), cardiac MRI, cardiac CT, coronary angiography, right heart catheter, endomyocardial biopsy, chest X-ray, thoracic and/or abdominal ultrasound, spiroergometry, and the 6-min-walk-test.

Preferably, the use of the polynucleotide for treatment and/or prevention of heart failure comprises administration of the polynucleotide systemically or to the heart of a subject. Systemic administration may be intravenous administration; cardiac administration may be administration to at least one coronary arteria and/or coronary vein, intramyocardial administration, pericardial administration, and/or atrial and/or ventricular administration. Administration may preferably be performed by ultrasound targeted microbubble destruction. More preferably, said use comprises intravenous administration of from $10^6$ to $10^{15}$, preferably of from $10^8$ to $10^{13}$, viral particles comprising said polynucleotide to said subject; preferably, said viral particles are AAV viral particles comprising the polynucleotide as specified herein above. Also preferably, the subject further receives or is planned to further receive additional therapeutic measures for treating and/or preventing heart failure or one of its comorbidities, in particular at least one therapy selected from pharmacological therapy, implantable cardioverter defibrillator (ICD) therapy, cardiac resynchronization therapy (CRT), ventricular assist device (VAD) therapy, and heart transplantation.

The use of the polynucleotide for treatment and/or prevention of heart failure further comprises administration of an RXFP1 agonist. The term "RXFP1 agonist" is understood by the skilled person to relate to a compound inducing RXFP1 signaling in cells comprising an RXFP1, preferably wherein said RXFP1 is expressed from a polynucleotide as specified herein. RXFP1 agonists are known in the art and include in particular ((phenylcarbamoyl)phenyl)benzamide-derivatives (Hu et al. (2016), Biochemistry 55(12):1772), in particular ML290 (2-Isopropoxy-N-(2-(3-(trifluoromethylsulfonyl) phenylcarbamoyl)phenyl)benzamide, CAS No: 1482500-76-4); or is Complement C1q tumor necrosis factor-related protein 8 (CTRP8, Genbank Acc No. NP_997302.2) Preferably, the RXFP1 agonist is a relaxin as specified herein above.

Advantageously, it was found that administration of a polynucleotide mediating expression of an RXFP receptor in heart tissue, in particular in the ventricles, provides for a significant improvement of heart failure, in particular in combination with concomitant administration of a relaxin. Moreover, the combination of administration of a polynucleotide mediating expression of an RXFP receptor in heart tissue with concomitant administration of a relaxin enables control over the genetic therapy after its administration by administration / non-administration of the agonist.

The definitions made above apply mutatis mutandis to the following. Additional definitions and explanations made further below also apply for all embodiments described in this specification mutatis mutandis.

The present invention also relates to a vector comprising the polynucleotide of the present invention for use in treatment and/or prevention of heart failure according to claim 11.

The term "vector" is specified herein above. Preferably, the vector has a tropism for cardiomyocytes as specified herein above. Also preferably, the vector is a viral vector, preferably an adeno-associated virus (AAV) vector as specified herein above.

The present invention further relates to a host cell comprising the polynucleotide of the present invention, an RXFP polypeptide expressed from a polynucleotide of the present invention, and/or the vector of the present invention for use in treatment and/or prevention of heart failure according to claim 12.

The present invention further relates to an RXFP1 agonist for use in the treatment of heart failure in a subject according to claim 13; said treatment comprises administration of a polynucleotide of the present invention, a vector of the present invention, and/or a host cell of the present invention, to said subject, preferably said treatment is preceded by the administration of a polynucleotide of the present invention, a vector of the present invention, and/or a host cell of the present invention to said subject.

Further disclosure not encompassed by the wording of the claims relates to a kit comprising a polynucleotide of the present invention, a vector of the present invention, and/or a host cell of the present invention, and a pharmaceutical preparation comprising an RXFP agonist.

The term "kit", as used herein, refers to a collection of the aforementioned compounds, means or reagents which may or may not be packaged together. The components of the kit may be comprised by separate vials (i.e. as a kit of separate parts) or provided in a single vial.

Moreover, it is to be understood that the kit of the present invention, preferably, is to be used for practicing the methods referred to herein above. It is, preferably, envisaged that all components are provided in a ready-to-use manner for practicing the methods referred to above. Further, the kit, preferably, contains instructions for carrying out said methods. The instructions can be provided by a user's manual in paper or electronic form. In addition, the manual may comprise instructions for administration and/or dosage instructions for carrying out the aforementioned methods using the kit of the present invention. As will be understood from the above, the description of the kit comprising polynucleotides, preferably, relates to a kit comprising corresponding vectors mutatis mutandis.

The term "pharmaceutical preparation", as used herein, relates to a preparation comprising the compound or compounds of the present invention in a pharmaceutically acceptable form and a pharmaceutically acceptable carrier. As will be understood by the skilled person, the polynucleotide, the vector, and the host cell as specified herein are, preferably, also provided as a pharmaceutical preparation as specified herein. The compounds of the present invention can be formulated as pharmaceutically acceptable salts. Acceptable salts comprise acetate, HCl, sulfate, chloride and the like. The pharmaceutical preparations are, preferably, administered topically or systemically. Suitable routes of administration conventionally used for drug administration are oral, intravenous, or parenteral administration as well as inhalation. Preferably, the pharmaceutical preparation of the present invention is administered via a parenteral route, preferably by intravenous, intraarterial, and/or intracardial administration. Moreover, the compounds can be administered in combination with other drugs either in a common pharmaceutical preparation or as separated pharmaceutical preparations wherein said separated pharmaceutical preparations may be provided in form of a kit of parts. As will be understood by the skilled person, the polynucleotides, vectors, host cells, and RXFP agonists may be administered via different routes even if administered to the same subject; e.g., preferably, a polynucleotide of the present invention may be administered intracardially, while concomitant or following administration of an RXFP agonist may be intravenously or subcutaneously.

The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are preferably selected so as not to affect the biological activity of the polynucleotide, vector, or host cell and potential further pharmaceutically active ingredients. Examples of such diluents are distilled water, physiological saline, Ringer's solutions, dextrose solution, and Hank's solution. In addition, the pharmaceutical preparation or formulation may also include other carriers, adjuvants, or nontoxic, nontherapeutic, nonimmunogenic stabilizers and the like.

A therapeutically effective dose refers to an amount of the compounds to be used in a pharmaceutical preparation of the present invention which prevents, ameliorates or treats a condition referred to herein. Therapeutic efficacy and toxicity of compounds can be determined by standard pharmaceutical procedures in cell culture or in experimental animals, e.g., by determining the ED50 (the dose therapeutically effective in 50% of the population) and/or the LD50 (the dose lethal to 50% of the population). The dose ratio between therapeutic and toxic effects is the therapeutic index, and it can be expressed as the ratio, LD50/ED50.

The dosage regimen will be determined by the attending physician, preferably taking into account relevant clinical factors and, preferably, in accordance with any one of the methods described elsewhere herein. As is well known in the medical arts, a dosage for any one patient may depend upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 μg to 10000 μg; however, doses below or above this exemplary range are envisioned, especially considering the aforementioned factors. Depending on the subject and the mode of administration, the quantity of substance administration may vary over a wide range to provide from about 0.01 mg per kg body mass to about 1 mg per kg body mass, preferably. The pharmaceutical preparations and formulations referred to herein are administered at least once in order to treat or prevent a disease or condition recited in this specification. However, the said pharmaceutical preparations may be administered more than one time; e.g. the polynucleotide, vector, and/or host cell may preferably be administered once, while the RXFP agonist may be administered several times, e.g. twice or three times, over a period of e.g. one week.

Specific pharmaceutical preparations are prepared in a manner well known in the pharmaceutical art and comprise at least an RXFP agonist and/or a polynucleotide, vector, and/or host cell as an active compound in admixture or otherwise associated with a pharmaceutically acceptable carrier or diluent. For making those specific pharmaceutical preparations, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescriber or user instructions in order to anticipate dose adjustments depending on the considered recipient.

A further disclosure not encompassed by the wording of the claims relates to a device comprising a polynucleotide of the present invention and/or a vector of the present invention.

The term "device", as used herein relates to a system of means comprising at least the means operatively linked to each other as to allow administration of the compound or of the composition of the present invention. Preferred means for administering polynucleotides, compositions, and/or host cells are well known in the art. How to link the means in an operating manner will depend on the type of means included into the device and on the kind of administration envisaged. Preferably, the means are comprised by a single device in such a case. Said device may accordingly include a delivery unit for the administration of the compound or composition and a storage unit for storing said compound or composition until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or composition of the invention. In another preferred embodiment, the device is an intravenous infusion (IV) equipment comprising the compound or composition. In another preferred embodiment, the device is a tube or an endoscopic device comprising the compound or medicament for flushing a site of administration, e.g. a heart, or further comprising a needle for topical application of the compound or composition, e.g. to a heart.

A further disclosure not encompassed by the wording of the claims relates to a method for treating heart failure in a subject, comprising i) contacting said subject with a polynucleotide of the present invention, a vector of the present invention, and/or a host cell of the present invention, and ii) thereby treating heart failure.

The method of treating of the present invention is an in vivo method. Moreover, it may comprise steps in addition to those explicitly mentioned above. For example, further steps may relate, e.g., to administration of an RXFP agonist concomitant to or following step i), and/or providing additional therapeutic measures for treating heart failure and/or one of its comorbidities. Moreover, one or more of said steps may be performed by automated equipment.

Further disclosure not encompassed by the wording of the claims relates to a use of a polynucleotide of the present invention, a vector of the present invention, and/or a host cell of the present invention for manufacturing a medicament for the treatment and/or prevention of heart failure.

Figure Legends

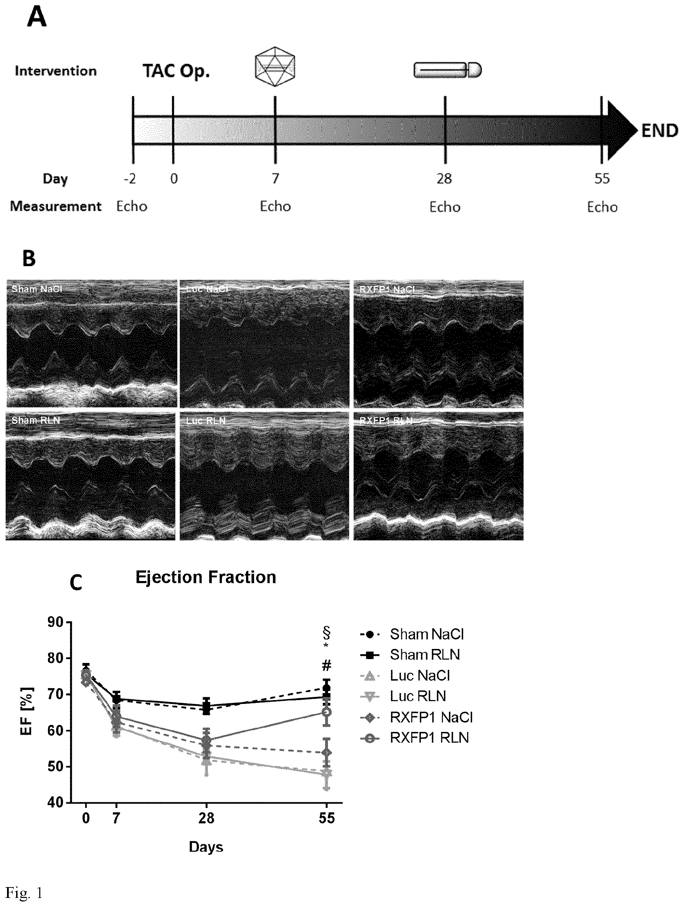

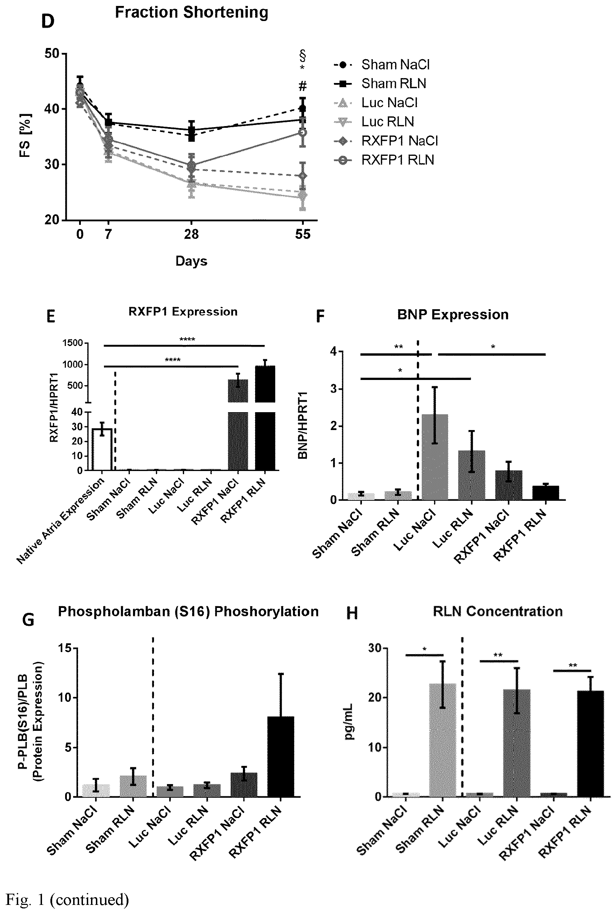

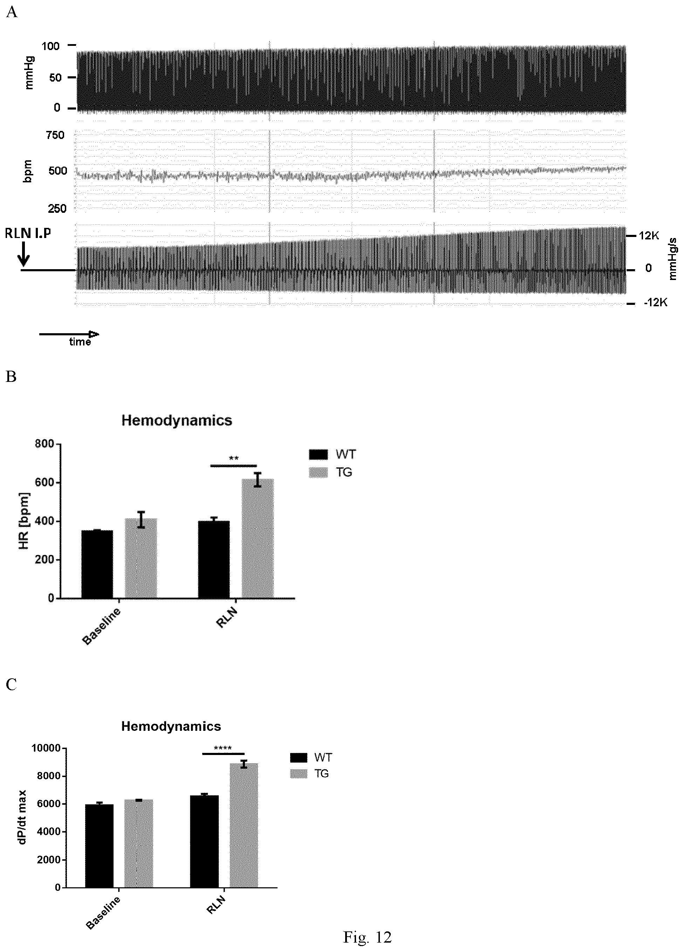

Fig. 1: RXFP1 gene therapy with chronic administration of RLN (RXFP1/RLN) improves cardiac function in TAC-induced heart failure in mice. (A) Experimental design. (B) Representative echocardiography recordings from midventricular M-Mode used for assessment of cardiac function. (C) Ejection Fraction (EF) and (D) fractional shortening (FS) of sham mice treated with and without recombinant RLN and TAC mice treated with control Luc virus and RXFP1 virus with and without recombinant RLN treatment (Sham + NaCl, Sham + RLN, TAC + LUC + NaCl, TAC + LUC + RLN, TAC + RXFP1 + NaCl and TAC + RXFP1 +RLN, n = 8-9 in sham groups and n = 14-15 in TAC groups). TAC animals were injected with $1 \times 10^{12}$ vg/animal and implanted with either NaCl pumps or RLN pumps 4 weeks after TAC operation. (E) RXFP1 mRNA expression in the ventricle detected 8 weeks after RXFP1 gene therapy treatment. RXFP1 mRNA expression from the treated samples were compared to the native RXFP1 expression in the atria. (F) BNP expression quantification by qPCR, HPRT1 was used as a reference gene. (G) cardiac P-PLB(S16)/PLB quantification by immunoblot; glyceraldehyde-3-phosphate dehydrogenase (GAPDH) immunodetection was used as an internal control. (H) Relaxin plasma concentrations were determined using an Elisa kit. For (C) and (D) * FL NaCl vs. FR RLN, # FL RLN vs.FR RLN § FR NaCl vs. FR RLN, */#/§ P< 0.05, for (E) to (H) *P < 0.05; **P < 0.01; ***P < 0.001

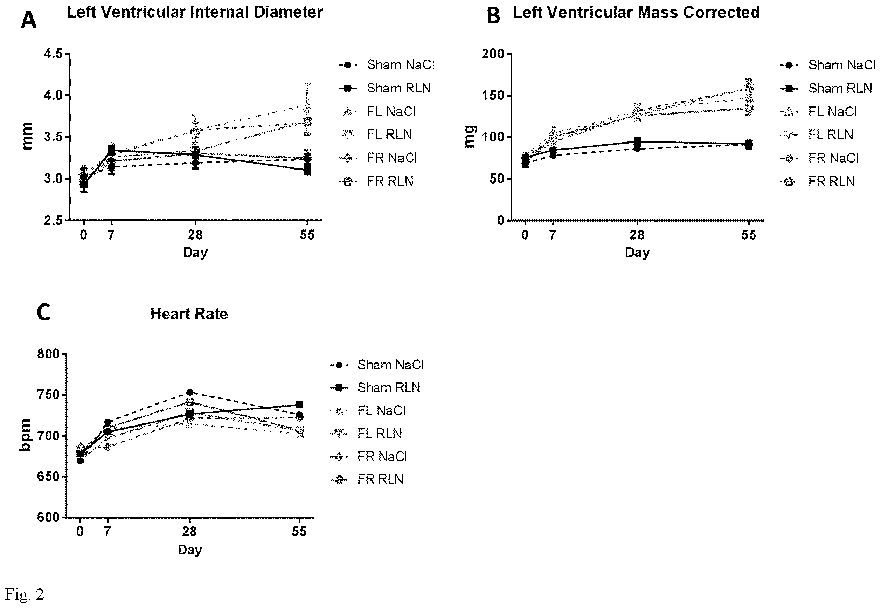

Fig. 2: RXFP1 gene therapy with chronic administration of RLN (RXFP1/RLN) maintains left ventricular internal diameter, but does not affect left ventricular mass and heart rate in TAC-induced heart failure in mice. TAC animals were injected with $1 \times 10^{12}$ vg/animal and implanted with either NaCl pumps or RLN pumps 4 weeks after TAC operation. * FL NaCl vs. FR RLN; # FL RLN vs.FR RLN; § FR NaCl vs. FR RLN (A) Left ventricular internal diameter (LVIdD) of sham mice treated with and without recombinant RLN and TAC mice treated with control Luc virus and RXFP1 virus with and without recombinant RLN (Sham + NaCl, Sham + RLN, TAC + LUC + NaCl, TAC + LUC + RLN, TAC + RXFP1 + NaCl and TAC + RXFP1 +RLN, n = 8-9 in sham groups and n = 14-15 in TAC groups); (B) Left ventricular (LV) mass of sham mice treated with and without recombinant RLN and TAC mice treated with control Luc virus and RXFP1 virus with and without recombinant RLN (Sham + NaCl, Sham + RLN, TAC + LUC + NaCl, TAC + LUC + RLN, TAC + RXFP1 + NaCl and TAC + RXFP1 +RLN, n = 8-9 in sham groups and n = 14-15 in TAC groups); (C) Heart rate of sham mice treated with and without recombinant RLN and TAC mice treated with control Luc virus and RXFP1 virus with and without recombinant RLN (Sham + NaCl, Sham + RLN, TAC + LUC + NaCl, TAC + LUC + RLN, TAC + RXFP1 + NaCl and TAC + RXFP1 +RLN, n = 8-9 in sham groups and n = 14-15 in TAC groups). */#/§ P< 0.05

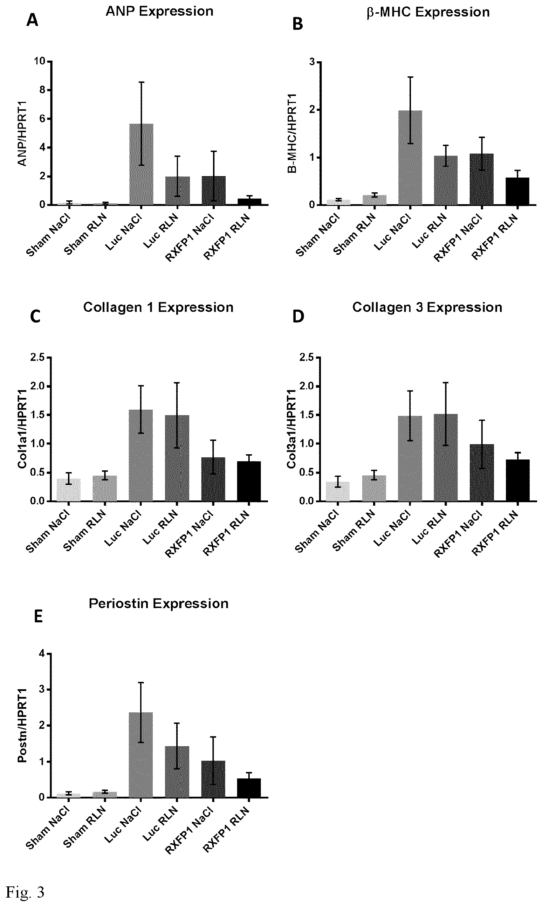

Fig. 3: Effect of RXFP1 gene therapy with chronic administration of RLN (RXFP1/RLN) on cardiac gene expression in TAC-induced heart failure in mice. Cardiac remodeling genes (A) ANP (B) β-MHC and collagen regulation genes (C) Col1a1 (D) Col3a1 (E) Postn mRNA expression in TAC animals receiving RXFP1 gene therapy with and without RLN quantified by qPCR with HPRT1 as reference gene. (n=8-15). *P < 0.05; **P < 0.01; ***P < 0.001; B)

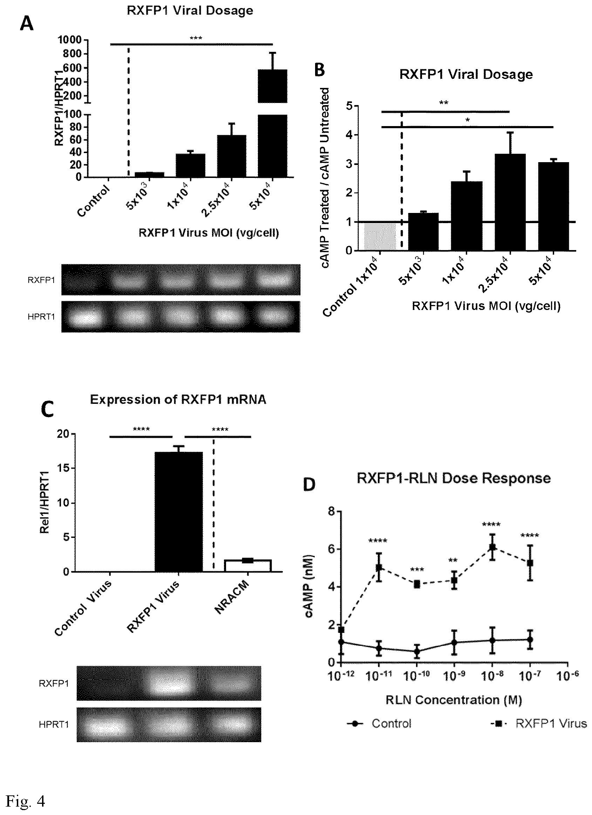

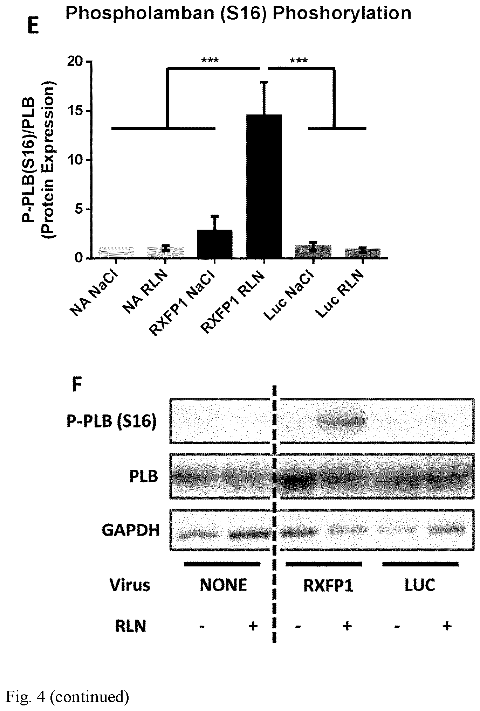

Fig. 4: Overexpression of RXFP1 in combination with RLN stimulation induces cAMP-dependent signaling in vitro; (A) RXFP1 mRNA expression in transduced NRVMs, n=3 per condition. *P < 0.05; **P < 0.01; ***P < 0.001; (B) cAMP production in transduced NRVMs, 100 nM ofRLN was used for RXFP1 stimulation under all conditions, n=3. *P < 0.05; **P < 0.01; ***P < 0.001; (C) RXFP1 mRNA expression in transduced NRVMs quantified by qPCR with HPRT1 as reference gene (n=6), control virus and RXFP1 virus were deployed in NRVMs, non-treated neonatal rat atrial cardiomyocytes (NRAM) were used as endogenous reference, *P < 0.05; **P < 0.01; ***P < 0.001; (D) RXFP1-RLN dose response as assessed by cAMP production, NRVMs were transduced with RXFP1 or control virus and stimulated with different dosages of RLN ranging from 1 pM to 100 nM and changes in cAMP production were measured, n=4-6, *P < 0.05; **P < 0.01; ***P < 0.001; (E-F) Characterization of cAMP-dependent downstream signaling, NRVMs were transduced with RXFP1 or Luc control virus and stimulated with 100 nM RLN or NaCl as control, phospholamban phosphorylation at serine 16 in relation to total phosholamban expression (P-PLB(S16)/PLB) was quantified by immunobloting, glyceraldehyde-3-phosphate dehydrogenase (GAPDH) was used as an internal control, (E) quantitative results, (F) representative immunoblots, n=4, *P < 0.05; **P < 0.01; ***P < 0.001.

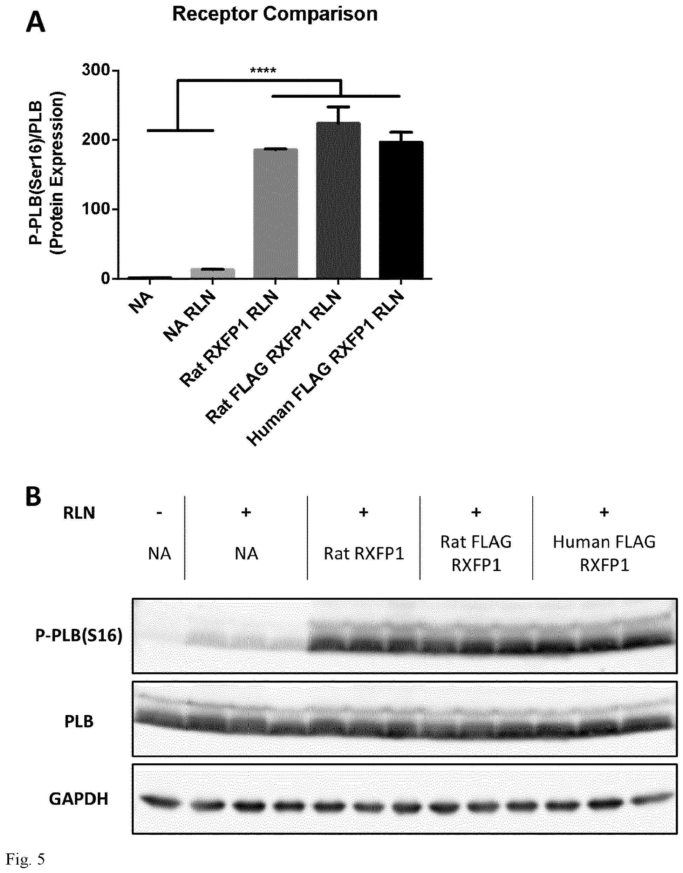

Fig. 5: Comparison of human and rat RXFP1 receptor activation by recombinant RLN.; A) P-PLB(S16)/PLB and (B) representative western blot of NRVMs transduced with different versions of RXFP1 receptor virus and stimulated by recombinant RLN (n=3), transduced NRVMs was stimulated with 100 nM recombinant RLN and phospholamban phosphorylation at serine 16 was quantified by immunoblot; glyceraldehyde-3-phosphate dehydrogenase (GAPDH) immunodetection was used as an internal control. *P < 0.05; **P < 0.01; ***P < 0.001.

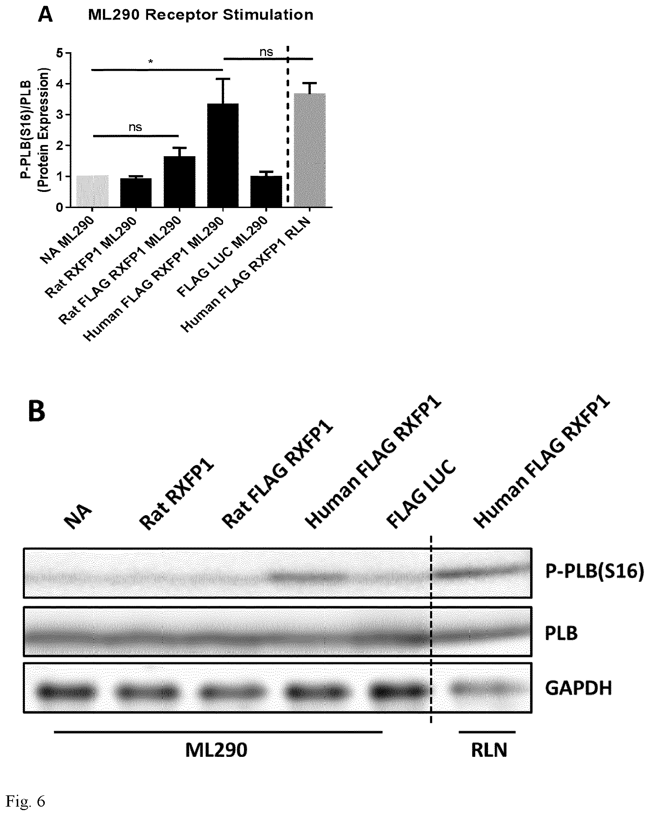

Fig. 6: Activation of human and rat RXFP1 receptor by a chemical agonist (ML290); (A) P-PLB(S16)/PLB in NRVMs transduced with different versions of RXFP1 receptor virus and stimulated by ML290 (n=3), transduced NRVMs were stimulated with 1 $\mu$M ML290 and phospholamban phosphorylation at serine 16 was quantified by immunoblot; glyceraldehyde-3-phosphate dehydrogenase (GAPDH) immunodetection was used as internal control. *P < 0.05; **P < 0.01; ***P < 0.001 (B) Western blot of P-PLB(S16)/PLB in NRVMs transduced with different versions of RXFP1 receptor virus and stimulated by ML290 (n=3), transduced NRVMs were stimulated with 1 $\mu$M ML290 and phospholamban phosphorylation at serine 16 was quantified by immunoblot; glyceraldehyde-3-phosphate dehydrogenase (GAPDH) immunodetection was used as an internal control. *P < 0.05; **P < 0.01; ***P < 0.001.

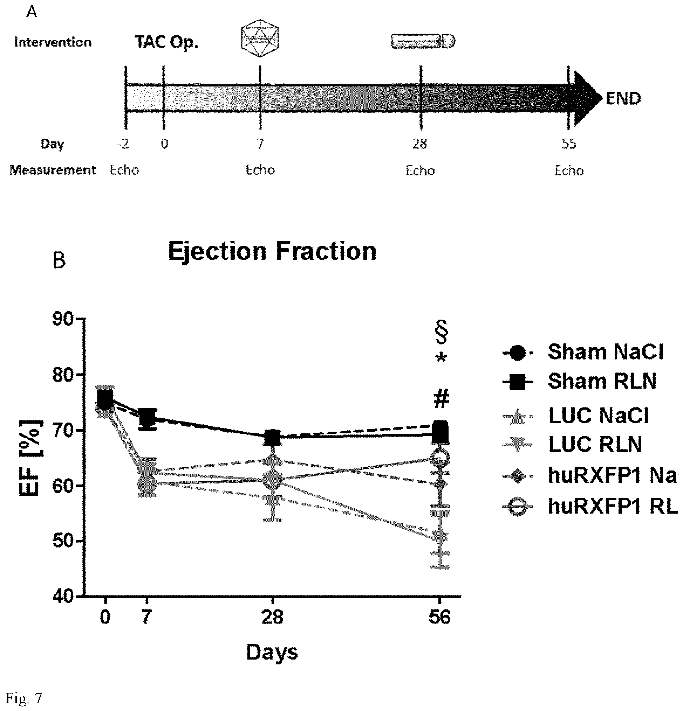

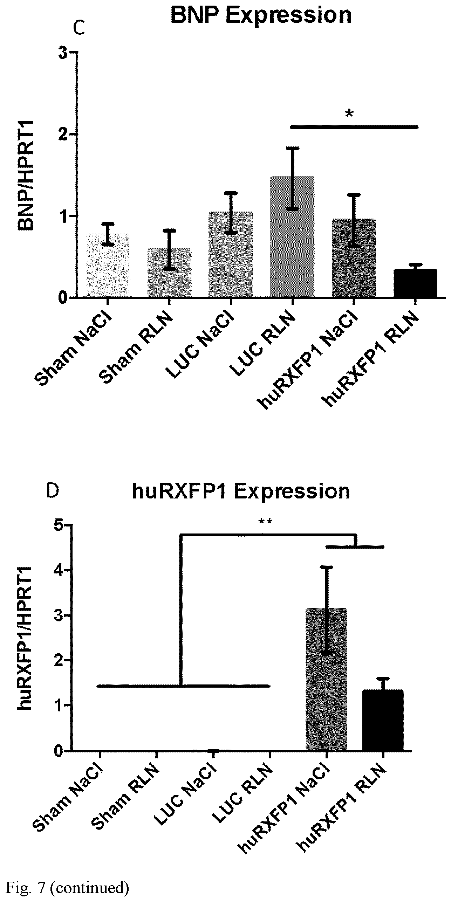

Fig. 7: Functional and molecular improvements after huRXFP1 gene therapy treatment with RLN: (A) Timeline of huRXFP1 study. (B) Ejection fraction (EF), of sham mice treated with and without recombinant RLN treatment and TAC mice treated with and without huRXFP1 gene therapy and RLN (Sham + NaCl, Sham + RLN, TAC + LUC + NaCl, TAC + LUC + RLN, TAC + huRXFP1 + NaCl and TAC + huRXFP1 + RLN, n=15 in sham groups and n=15-22 in TAC groups). TAC animals were injected with 1x10$^{12}$ vg/animal and implanted with either NaCl pumps or RLN pumps 4 weeks after TAC operation. (C) BNP and (D) huRXFP1 mRNA expression in the ventricle detected 8 weeks after huRXFP1 gene therapy treatment (n=15 in sham groups and n=15-22 in TAC groups). mRNA expression was quantified using qPCR, with HPRT1 as a reference gene. For (B) * FL NaCl vs. huRXFP1 RLN, # LUC RLN vs. huRXFP1 RLN § huRXFP1 NaCl vs. huRXFP1 RLN, for (C) and (D) *P < 0.05; **P < 0.01; ***P < 0.001. Error bars depict in SEM.

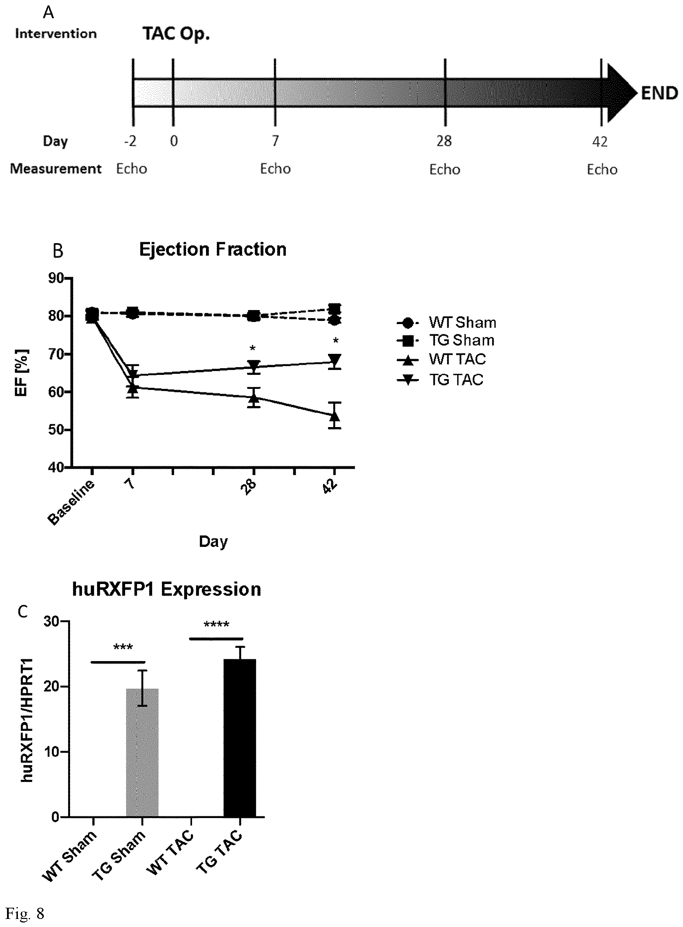

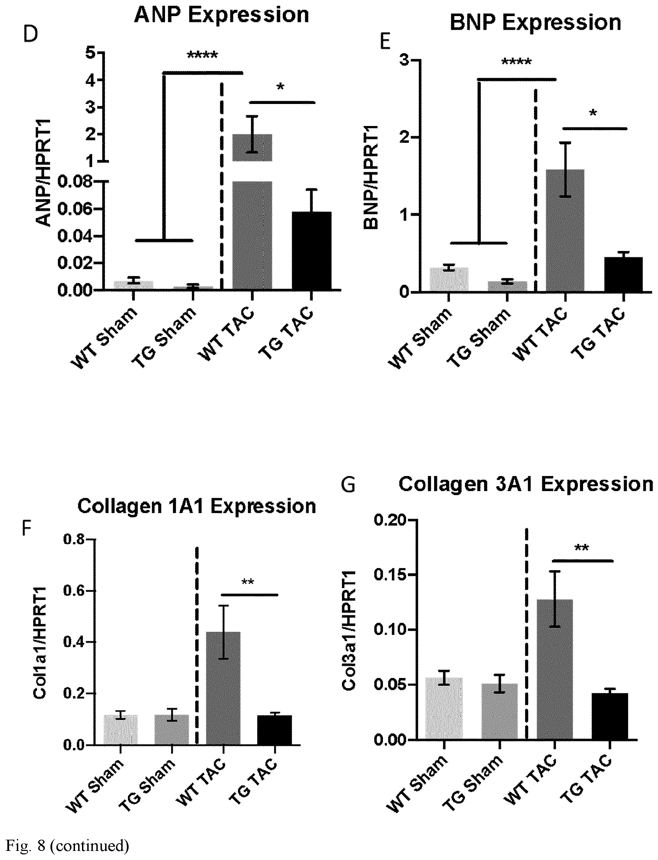

Fig. 8: Functional and molecular improvements in stress model with high expression of huRXFP1. (A) Timeline of the experiment. (B) Ejection fraction (EF) of TAC transgenic animals (n=10-20). After TAC operation, the animals were periodically followed using echocardiography. (C) huRXFP1, (D) ANP, (E) BNP, (F) Collagen1A1, and (G) Collagen1A3 mRNA expression in the ventricle detected 6 weeks after the TAC operation (n=10-20). mRNA expression was quantified using qPCR, with HPRT1 as a reference gene. For (B) * TAC TG vs TAC WT, for (C-G) *P < 0.05; **P < 0.01; ***P < 0.001. Error bars depict in SEM.

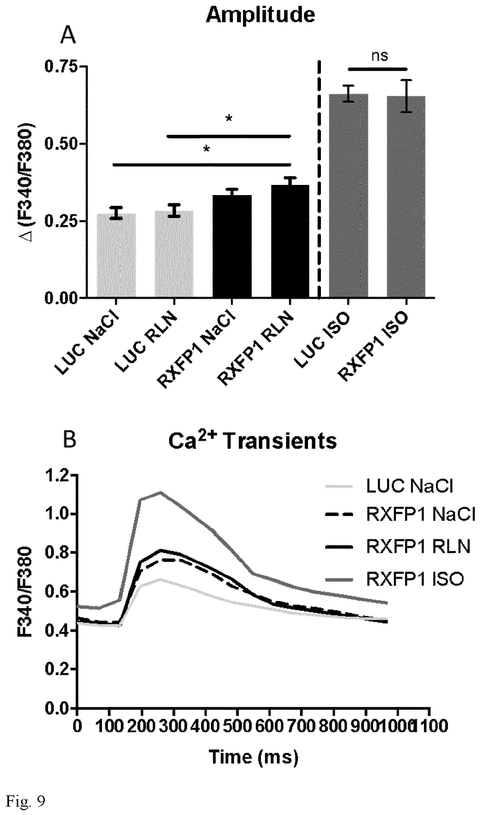

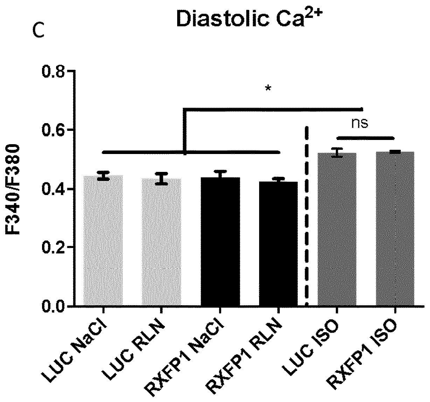

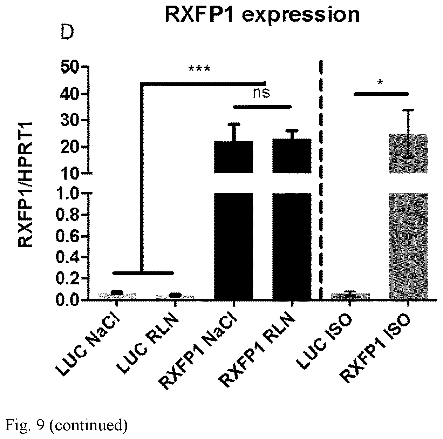

Fig. 9: Calcium transients measurement in RXFP1 transduced NRVCMs: (A), (B) Transients amplitude and representative Ca$^{2+}$ transients and (C) Diastolic Ca$^{2+}$ after 5 minutes of NaCl, RLN, and ISO treatments (n=3-8). NRVCMs were transduced with RXFP1 and LUC virus for 5 days. Ca$^{2+}$ transients were measured with 1 Hz electrical stimulation. (D) RXFP1 mRNA expression after the experiment. RXFP1 mRNA expression was analyzed using HPRT1 as a reference gene. For figure A only the first 4 groups were compared. *P < 0.05; **P < 0.01; ***P < 0.001. Error bars depict in SEM.

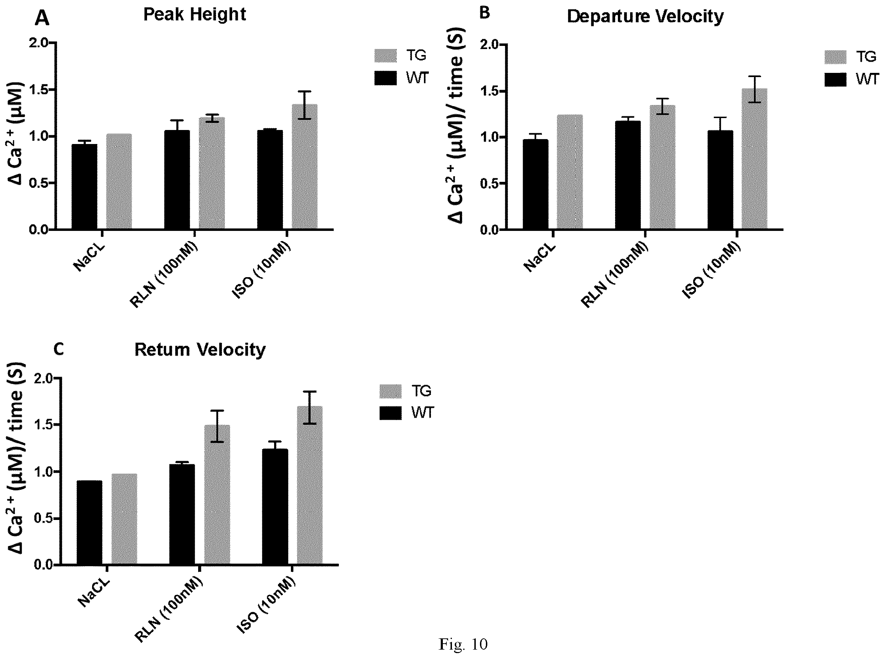

Fig. 10: Adult cardiomyocyte Ca$^{2+}$ transient: (A) Ca$^{2+}$ peak height, (B) Departure velocity, (C) Return velocity after RLN treatment (n=2). Adult cardiomyocytes were isolated from transgenic mice that expressed RXFP1 specifically in the heart. Baseline Ca$^{2+}$ was measured. The cells were treated with NaCl, RLN (100 nM), or ISO (10 nM) for 1 minute and the transients were measured again. Alteration of Ca$^{2+}$ signal was calculated using automatic software and normalized against baseline value. *P < 0.05; **P < 0.01; ***P < 0.001. Error bars depict in SEM.

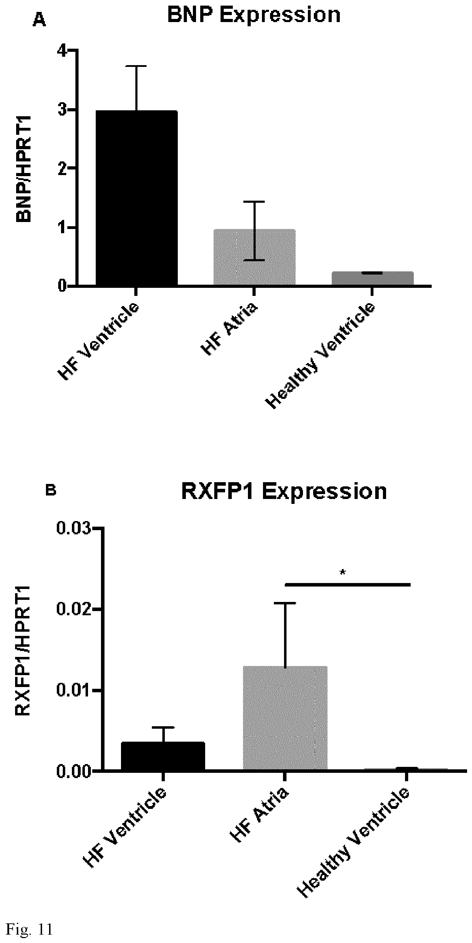

Fig. 11: RXFP1 expression in heart failure: (A) BNP mRNA expression. (B) RXFP1 mRNA expression. mRNA expression profile of fetal gene and RXFP1 from explanted hearts and cardiac biopsies were performed. mRNA expression was quantified using qPCR, with HPRT1 as a reference gene. *P < 0.05; **P < 0.01; ***P < 0.001. Error bars depict in SEM.

Fig. 12: Figure 12: Hemodynamics changes in transgenic animals after RLN administration (Example 10). (A) Representative PV loops recording, (B) Heart Rate (HR), and (C) dP/dt maximum. Continuous PV loops were measured from WT and huRXFP1 TG animals. 10 $\mu$g in 100 $\mu$L of RLN was administered to stimulate huRXFP1. *P < 0.05; **P < 0.01; ***P < 0.001; ****P < 0.0001. Error bars depict in SEM.

The following Examples shall merely illustrate the invention. They shall not be construed, whatsoever, to limit the scope of the invention.

***Example 1: RXFP1 gene therapy in combination with RLN administration induces positive inotropic effects and rescues heart failure***

**Virus Production**

For all *in vivo* experiments adeno-associated viral vectors serotype 9 (AAV9) were generated. Recombinant AAV vectors expressing rat *(Rattus norwegicus)* and human *(Homo sapiens)* relaxin receptor 1 (RXFP1) were generated: AAV9-CMV-MLC260-FLAG-*Rel1*Rattus (AAV9-RXFP1) and AAV9-CMV-MLC260-FLAG-*Rel1*human (AAV9-huRXFP1). Both optimized rat and human *Rel1* cDNA (NM_201417.1 and NM_021634.3, National Center for Biotechnology Information (NCBI)) were synthesized and cloned into a pCDNA3.1 plasmid with N-terminal FLAG-TAG. After extensive *in vitro* testing, the transgene cassette was subcloned into an AAV transfer plasmid downstream of a cardiac myocyte-specific cytomegalovirus (CMV) enhancer coupled with a short 260bp myosin light chain promoter (MLC260) and the whole construct was flanked by two ITRs. Recombinant vectors were generated by packaging of AAV-inverted terminal repeat recombinant genomes into AAV9 capsids using the two plasmids transfection protocol (Grimm et al., 2003, Mol. Ther. 7: 839-850). High titer vectors were produced in cell stacks (Corning) with polyethylenimine transfection. After 48 hours the vectors were harvested and purified by density filtration in an iodixanol gradient (Jungmann et al., 2017, Hum. Gene. Ther. Methods 28: 235-246). Using the same method, a control vector expressing firefly luciferase was generated: AAV9-CMV-MLC260-FLAG-*Luc*Firefly (AAV9-LUC). Viral titers from all viral vectors were quantified at the same time using a SYBR-green real time PCR assay (Bio-Rad) and expressed as viral genomes per milliliter (vg/mL).

**Heart failure model and experimental set up**

Transverse aortic constriction (TAC) operation was previously described (Rockman et al., 1991, Proc. Natl. Acad. Sci. 88: 8277-8281). TAC model was used as heart failure model. 8 weeks old C57BL/6 mice were weighted prior to the operation. TAC operation was performed on day 0 using 26-gauge blunt needle effectively reducing the diameter of the transverse aorta to approximately 0.46 mm (Rockman et al., 1994, Proc. Natl. Acad. Sci. 91(7): 2694-2698). Cardiac function was assessed by echocardiography (Vevo 2100 VisualSonics) before TAC operation (day -2), before virus infusion (day 7), before pump implantation (day 28), and at 2-month follow up (day 55) (Fig. 1A and B). At day 7, animals were randomized to systemic injection of luciferase (LUC) control virus or RXFP1 virus. Osmotic pumps (Alzet 2004) were implanted on day 28 with animals randomized to get either saline or recombinant relaxin pumps. Follow up assessments were performed on day 55 and the animals were sacrificed 3 days later. In addition, 17 mice served as control animals which underwent sham operation, followed by either saline or recombinant relaxin pump implantation.

**Cardiac function measurements**

Echocardiography was performed using Vevo 2100, VisualSonics. The mice were shaved and held in prone position. Warm ultrasound coupling gel (37°C) was placed on the shaved chest area, and the MS400 transducer was positioned to obtain 2D B-mode parasternal long and short axis views and M-mode short axis view. The ejection fraction (EF), fractional shortening (FS), left ventricular internal diameter (LVIDd) and heart rate (HR) were calculated from 6 consecutive heartbeats in the M-mode using LV trace function.

EF and FS of all TAC animals gradually decreased starting from day 7 post TAC operation (EF: $62.25 \pm 10.41\%$ and FS: $33.17 \pm 7.38$). Further reduction was detected in all TAC operated animals on day 28 post operation before pump implantation (EF: $54.09 \pm 12.65$ and FS: $28.07 \pm 7.63$). The follow up assessments at day 55 showed further reduction in EF and FS in control groups treated with AAV9.LUC receiving either NaCl or RLN (LUC NaCl EF: $48.78 \pm 18.61$; FS: $25.11 \pm 10.69$ and LUC RLN EF: $47.79 \pm 13.38$; FS: $23.98 \pm 7.64$). An attenuatedhowever non-significant decrease in EF and FS was observed in RXFP1 groups treated with NaCl (EF: $53.99 \pm 13.75$ and FS: $27.95 \pm 8.40$) and a significant increase in EF and FS was detected in RXFP1 groups treated with RLN (EF: $65.11 \pm 14.39$ and FS: $35.74 \pm 9.92$) (Fig. 1C and D). Other parameters such as left ventricular internal diameter (LVIdD), left ventricular mass corrected, and heart rate were also measured, but no significant difference between TAC operated groups could be observed (Fig. 2A, B, and C).

**Molecular Analysis**

**RNA expression**

RNA was extracted using TRIzol® reagent (Ambion) from snap frozen tissue (10mg) or cell lysate ($2\times10^6$ cells) according to the manufacturer's protocols. 1 μg of total the RNA was reverse transcribed into cDNA using the iScript cDNA-Synthesis Kit (Bio-Rad). iQ SYBR Green Supermix was used according to the manufacturer's protocol. A final

volume of 15 μL per reaction consisted of 7.5 μL iQ SYBR Green Supermix, 1 μL of forward and reverse primers mix (final concentration of 300 nM each), and 6.5 μL diluted cDNA prepared (final concentration of 3.25 ng per reaction). The quantitative real-time PCR was performed in duplicate on Bio-Rad CFX96 real-time PCR detection system (Bio-Rad). The $2^{-\Delta\Delta CT}$ method was used to quantify relative gene expression levels between samples. Specificity of PCR products was confirmed by gel electrophoresis. Primers listed in table 1 were used for mRNA expression quantification.

**Table 1:** Primers for RT-PCR

| Genes | Primers | Template | SEQ ID NO |
|---|---|---|---|
| *ANP* | Forward primer: 5'-TGCCGGTAGAAGATGAGGTC-3' | NM_008725.3 | 14 |
| | Reverse primer: 5'-TGCTTTTCAAGAGGGCAGAT-3' | | 15 |
| *β-MHC* | Forward primer: 5'-GCCAACACCAACCTGTCCAAGTTC-3' | NM_080728.3 | 16 |
| | Reverse primer: 5'-TGCAAAGGCTCCAGGTCTGAGGGC-3' | | 17 |
| *BNP* | Forward primer: 5'-CTGAAGGTGCTGTCCCAGAT-3' | NM_008726.5 | 18 |
| | Reverse primer: 5'-CCTTGGTCCTTCAAGAGCTG-3' | | 19 |
| *Col1a1* | Forward primer: 5'-GTGTTCCCTACTCAGCCGTC-3' | NM_007742.4 | 20 |
| | Reverse primer: 5'-ACTCGAACGGGAATCCATCG-3' | | 21 |
| *Col3a1* | Forward primer: 5'-TGACTGTCCCACGTAAGCAC-3' | NM_009930.2 | 22 |
| | Reverse primer: 5'-GAGGGCCATAGCTGAACTGA-3' | | |
| *Hprt1* | Forward primer: 5'-GAGGAGTCCTGTTGATGTTGCCAG-3' | NM_013556.2 | 24 |
| | Reverse primer: 5'-GGCTGGCCTATAGGCTCATAGTGC-3' | | 25 |
| *Postn* | Forward primer: 5'-ACAAAAGGGTTCAAGGGCCTA-3' | NM_009071.2 | 26 |
| | Reverse primer: 5'-TTGGCTTCTGTTGGTTGTCA-3' | | 27 |
| *Rattus Norvegicus Rel1* | Forward primer: 5'-CCATGCATTGTTTGTGCCGA-3' | NM_201417.1 | 28 |
| | Reverse primer: 5'-TTTGCAGGCACAGCTTTTGG-3' | | 29 |
| *Homo sapiens Rel1* | Forward primer: 5'-CTACAAGGACGACGATGACAAG-3' | NM_021634.3 | 30 |
| | Reverse primer: 5'-GAAATAGCCAAGGGAGCACTTG-3' | | 31 |

**Table 2:** RT-PCR program

| Step | Temperature | Time | Number of Cycles |
|---|---|---|---|
| Denaturation | 95° C | 3:00 | 1x |
| Denaturation | 95° C | 0:10 | |
| Annealing | Vary annealing Temperature | 0:10 | 40x |
| Elongation and reading | 72° C | 0:30 | |
| Termination | 95° C | 0:10 | 1x |
| Melting Curve | 65 → 95° C with 0.5° C increment | 0:05 | 60x |

Post-mortem mRNA expression analysis using RTPCR revealed a significant increase in RXFP1 mRNA expression within the ventricle of TAC animals treated with RXFP1 virus (Fig. 1E). Compared to a TAC group treated with control virus alone, a significant decrease in BNP mRNA expression was observed in RXFPI and RLN treated mice (Fig. 1F).

Furthermore, a trend towards reduced activation of fetal genes like *ANP* or *β-MHC* was detected in the TAC group receiving both RXFP1 gene therapy and RLN treatment (Fig. 3A and B). Interestingly, a trend toward decreased collagen mRNA expression (*Col1a1*, *Col3a1* and *Postn*) post TAC was detected in both RXFP1 groups (with and without RLN treatment) (Fig 3C, D and E).

## Protein Expression

**Table 3:** List of antibodies used for immunoblotting

| Protein | Manufacturer | Type | Species | Reactivity | Dilution |
|---|---|---|---|---|---|
| Phospholamban (PLB) | Sigma Aldrich | Monoclonal | Mouse | Mouse, Rat, Human | 1:7500 |
| Phospho (serine16)-phospholamban [P-PLB(S16)] | Upstate | Polyclonal | Rabbit | Mouse, Rat, Human | 1:5000 |
| Phosphor (threonine17)-phospholamban [P-PLB (T17)] | Badrilla | Polyclonal | Rabbit | Mouse, Rat, Human | 1:5000 |
| GAPDH | Merck-Millipore | Monoclonal | Mouse | Mouse and Rat | 1:10000 |

Tissue samples were cut into 10 mg pieces prior to freezing. Proteins from tissues were extracted using 1% SDS buffer (1%SDS, 1 mM EDTA, 1 mM EGTA supplemented with protease inhibitor and phosphatase inhibitor cocktail 2 and 3). Extracted proteins were analyzed by SDS-polyacrylamide gel electrophoresis and Western blotting as previously described (Towbin et al., 1979, Proc. Natl. Acad. Sci. 76: 4350-4354). Membranes were probed with antibodies listed in table 3. Mouse secondary antibody coupled to Alexa Flour 680 (Invitrogen) and rabbit secondary antibody coupled to Dylight™ 800 (Cell Signaling) were used to detect the signals from the immunoblots utilizing an Odyssey infrared imager (LI-COR) detection system. Images were processed using Odyssey imaging software. A higher ratio of phospholamban phosphorylation at serine 16 [P-PLB(S16)] was detected in TAC animals that received both RXFP1 gene therapy and RLN (Fig. 1G).

## RLN Measurements

Circulating plasma levels of recombinant relaxin H2 were measured using a ready to use relaxin H2 Quantikine ELISA kit from R&D Systems (DLR200). Plasma samples were diluted 1:200 to stay within the detection range of the assay. 50 μL of negative control, positive control, standard and diluted samples were used for the measurement.

Measurements of RLN plasma levels revealed elevated plasma levels of RLN in all animals receiving RLN. RLN plasma concentrations were equal in all treated groups (Fig. 1H).

***Example 2 Positive inotropy from RXFP1 gene therapy and recombinant RLN stimulation in vitro***

## Virus production

For all *in vitro* experiments AAV6 serotype vectors were deployed. The recombinant adeno-associated viral vectors AAV6-CMV-MLC260-FLAG-*Rel1*Rattus (AAV6-RXFP1) and AAV6-CMV-MLC260-FLAG-*Rel1*human (AAV6-huRXFP1) were generated as follows. Both optimized *Rattus Norvegicus* and *Homo sapiens RXFP1* cDNA [NM_201417.1 and NM_021634.3, National Center for Biotechnology Information (NCBI)] were synthesized and cloned into a pCDNA3.1 plasmid with N-terminal FLAG-TAG. After extensive *in vitro* testing, the transgene cassette was subcloned into an AAV transfer plasmid downstream of a cardiac myocyte-specific cytomegalovirus (CMV) enhancer coupled with a short 260bp myosin light chain promoter (MLC260) and the whole construct was flanked by two ITRs. Recombinant vectors were generated by packaging of AAV-inverted terminal repeat recombinant genomes into AAV6 capsids using the two plasmids transfection protocol (Grimm et al., 2003, Mol. Ther. 7: 839-850). High titer vectors were produced in cell stacks (Corning) with polyethylenimine transfection. After 48 hours the vectors were harvested and purified by density filtration in an iodixanol gradient (Jungmann et al., 2017, Hum. Gene. Ther. Methods 28: 235-246). Using the same method, the control vector AAV6-CMV-MLC260-FLAG-*Luc*Firefly (AAV6-LUC) was generated. Viral titers from all viral vectors were quantified at the same time using a SYBR-green real time PCR assay (Bio-Rad) and expressed as viral genomes per milliliter (vg/mL).

**Isolation of Neonatal Rat Cardiomyocytes**

Primary cultures of neonatal rat ventricular cardiac myocytes (NRVMs) were prepared from 1-2 days old Wistar rats (Charles River). The neonatal rats were euthanatized by decapitation and the heart dissected and placed in 1X cold ADS solution. The atria and other vessels were removed, and the hearts were transferred into a new 1X cold ADS solution. The ADS solution was removed, and the ventricles were washed with 3 mL of cold ADS before being minced. Tissue fragments were digested in a T75 bottle with 30 mL of a digestion solution containing collagenase and pancreatin for 5 minutes at 37° C. The tissue fragments were allowed to settle on one side of the bottle and the solution removed and discarded. Fresh digestion solution was added, and the tissue fragments were digested for 20 minutes at 37° C. After 20 minutes, the tissue fragments were pipetted up and down 5 times at maximum power and the solution removed and filtered through a 40 $\mu$m cell strainer into a 50 mL falcon. 10 mL of FCS was added through the cell strainer and the solution was centrifuged at 1000 rpm for 5 minutes at room temperature. After centrifugation, the supernatant was removed, and 7 mL of FCS was added. The cell solution was placed in a 37°C incubator awaiting further processing. The tissue fragments were digested at least 5 times in order to completely digest all the tissue pieces. After the digestion, all cell suspensions were combined and centrifuged at 1000 rpm for 5 minutes at room temperature. The supernatant was removed, and the cells were resuspended in 12 mL of cold 1X ADS. The fibroblasts and cardiac myocytes were separated using percoll density gradient. To obtain cardiomyocytes with high purity, a two layers Percoll density gradient was performed. The gradient consisted of 63% red Percoll solution, and a 40.5% clear Percoll solution. 4 mL of red Percoll solution was added into each 15 mL falcon. Then 3 mL of clear percoll solution was added using underlaying technique. 2 mL of cell solution containing 1X cold ADS was layered at a 45 degrees angle onto of the gradient. The falcons were centrifuged at 2400 rpm for 30 minutes at 37° C deceleration speed of 0. The upper stromal cells band including fibroblasts was removed by aspiration. The lower cardiomyocytes band was kept and washed twice with cold 1X ADS before being resuspended in a warm Medium 199 supplemented with 10% v/v fetal calf serum, 100 U/mL penicillin, 100 $\mu$g/ streptomycin, 2 mM L-glutamine, and 1 mM calcium chloride. The cells were counted manually in a Neubauer chamber and plated accordingly (Table 4). The NRVCM were cultured at 37° C and 5% $CO_2$ humidified atmosphere. After 48 hours fetal calf serum was reduced to 0.5% and cells were cultured for 2-5 days. Two days after the cells were plated, the medium was removed, and the cells were washed once with warm PBS before starving medium M199 supplemented with 0.5% FCS, 1% penicillin/streptomycin, 1% L-glutamine, 1 mM $CaCl_2$ was added. In the following, the medium was changed every two days until the cells were harvested. Cells were used for the maximum of 7 days before being discarded.

**Table 4:** Optimal plating density for NRVMs

| | Area per well (cm$^2$) | Number of cells seeded | Volume per well (mL) |
|---|---|---|---|
| 6-well plate | 10 | $2 \times 10^6$ | 2 |
| 12-well plate | 4 | $5 \times 10^5$ | 1 |
| 96-well plate | 0.32 | $2 \times 10^4$ | 0.1 |

**Optimization of neonatal rat ventricular cardiomyocyte transfection for *in vitro* testing of RXFP1 gene therapy**

After the first medium change NRVMs seeded in 6-well plates were transduced with different amounts of RXFP1 virus (ranging from viral MOI of 5,000 to 50,000 vg/cell) along with Luc control virus with the MOI of 10,000 vg/cell. The virus was allowed to transduce the cells for 48 hours before the medium was changed. After 5 days the transduced NRVMs were harvested and RXFP1 mRNA expression was quantified by qPCR using primers from table 1. Significant increase in RXFP1 mRNA expression could be detected in a dose dependent manner (Fig. 4A). To further confirm the optimal MOI, cAMP measurements were performed using a cAMP Glo™ assay kit from Promega. Manufacture's manual was followed. First NRVMs were seeded into a 96-well according to table 4. The cells were transduced with different MOI of RXFP1 virus ranging from MOI of 5,000 to 50,000 vg/cell and a fixed MOI of Luc control virus at 10,000 vg/cell. The virus was allowed to transduce the cell for 48 hours before the medium was changed. After 5 days the transduced NRVMs were treated with 100 nM of recombinant RLN for 30 minutes. To stimulate the traduced NRVMs, the medium was removed and 20 $\mu$L of RLN dissolved in induction buffer (1X PBS supplemented with 500 $\mu$M IBMX and 100 $\mu$M Ro 20-1724) was added to the cells to stimulate cAMP production. After 30 minutes, 20 $\mu$L of lysis buffer was added to all wells. The cells were lysed at room temperature for 15 minutes. 40 $\mu$L of detection buffer (Reaction buffer supplemented with Protein Kinase A) was added to all wells, and the plate was mixed by shaking for 1 minute and incubated at room temperature for 20 minutes. 80 $\mu$L of Kinase Glo® Reagent was added to all wells and the plate was mixed by shaking for 1 minute and incubated at room temperature for 10 minutes. The luminescence was measured with a luminometer. A significant increase in cAMP production was observed in samples that received at least 10,000 vg/cell RXFP1, but a decrease in cAMP production was detected with a MOI of 50,000 vg/cell (Fig. 2B). Thus, lowest viral MOI with both RXFP1 mRNA expression and significant increase in intracellular cAMP production following RLN stimulation was 10,000 vg/cell.

**Ectopic RXFP1 expression vs native RXFP1 expression**

NRVMs seeded in 6-well plates were transduced with RXFP1 virus and Luc control virus with the viral MOI of 10,000 vg/cell. The virus was allowed to transduce the cells for 48 hours before the medium was changed. After 5 days the transduced NRVMs were harvested and RXFP1 mRNA expression was quantified by qPCR using primers listed table 1. Significant increase in RXFP1 mRNA expression could be detected in RXFP1 virus treated cells compared to Luc control treated cells and untreated cells with native RXFP1 expression from the atria (neonatal rat atrial cardiomyocytes, NRAM) (Fig. 4C).

**Optimal concentration of RLN stimulation determined by cAMP accumulation**

cAMP measurement was performed using an cAMP Glo™ assay kit from Promega. Manufacture's manual was followed. First NRVCMs were seeded on a 96-well plate according to table 4. The cells were transduced with RXFP1 virus and Luc control virus with a viral MOI of 10,000 vg/cell. The virus was allowed to transduce the cell for 48 hours before the medium was changed. After 5 days the transduced NRVMs were treated with different amounts of recombinant RLN ranging from 1 pM to 10 nM for 30 minutes. To stimulate the tranduced NRVMs, the medium was removed and 20 $\mu$L of RLN in induction buffer (1X PBS supplemented with 500 $\mu$M IBMX and 100 $\mu$M Ro 20-1724) were added to the cells to stimulate cAMP production. After 30 minutes, 20 $\mu$L of lysis buffer was added to all wells. The cells were lysed at room temperature for 15 minutes. 40 $\mu$L of detection buffer (Reaction buffer supplemented with Protein Kinase A) was added to all wells, and the plate was mixed by shaking for 1 minute and incubated at room temperature for 20 minutes. 80 $\mu$L of Kinase Glo® Reagent was added to all wells and the plate was mixed by shaking for 1 minute and incubated at room temperature for 10 minutes. The luminescence was measured with a luminometer. The results confirmed that a substantial increase in cAMP production was observed in RXFP1 virus and recombinant RLN treated cells compared to cells treated with Luc control virus and recombinant RLN (Fig. 4D). Peak stimulation of the RXFP1 receptor was observed at a recombinant RLN concentration of 10 nM. Interestingly, no further increase in cAMP accumulation with higher recombinant RLN concentrations was found.

**Positive inotropy from RXFP1 gene therapy with recombinant RLN treatment**

NRVMs seeded in 6-well plates were transduced with RXFP1 virus and Luc control virus at a viral MOI of 10,000 vg/cell. The virus was allowed to transduce the cell for 48 hours before the medium was changed. After 5 days the transduced NRVMs were stimulated with 100 nM recombinant RLN for 5 hours before being harvested for immunoblotting using 1% SDS buffer (1%SDS, 1 mM EDTA, 1 mM EGTA supplemented with protease inhibitor and phosphatase inhibitor cocktail 2 and 3). Proteins were analyzed by SDS-polyacrylamide gel electrophoresis and immunoblotting as previously described (Towbin et al., 1979, Proc. Natl. Acad. Sci. 76: 4350-4354). Membranes were probed with antibodies listed in table 3. Mouse secondary antibody coupled to Alexa Flour 680 (Invitrogen) and rabbit secondary antibody coupled to Dylight™ 800 (Cell Signaling) were used to detect the signals from the immunoblots utilizing Odyssey infrared imager (LI-COR) detection system. Images were processed using Odyssey imaging software. A significant increase in P-PLB(S16) was observed in the NRVM groups transduced with RXFP1 virus and treated with recombinant RLN (Fig. 4E-F). In line with *in vivo* experiments, molecular evidences from the *in vitro* experiments suggest that positive inotropy is a result of increased cAMP production and increased phospholamban phosphorylation at serine 16.

***Example 3 Comparison of human and rat RXFP1 gene therapy***

**Virus production**

For all *in vitro* experiments AAV6 serotype vectors were deployed. AAV6-CMV-MLC260-*Rel1*^Rattus (designated AAV6-naRXFP1) was generated as follows. Optimized *Rattus Norvegicus Rel1* cDNA [NM_201417.1, National Center for Biotechnology Information (NCBI)] were synthesized and cloned into a pCDNA3.1 plasmid. After extensive *in vitro* testing, the transgene cassette was subcloned into an AAV transfer plasmid downstream of a cardiac myocyte-specific cytomegalovirus (CMV) enhancer coupled with a short 260bp myosin light chain promoter (MLC260) and the whole construct was flanked by two ITRs. Recombinant vectors were generated by packaging of AAV-inverted terminal repeat recombinant genomes into AAV6 capsids using the two plasmids transfection protocol (Grimm et al., 2003, Mol. Ther. 7: 839-850). High titer vectors were produced in cell stacks (Corning) with polyethylenimine transfection. After 48 hours the vectors were harvested and purified by density filtration in an iodixanol gradient (Jungmann et al., 2017, Hum. Gene. Ther. Methods 28: 235-246). Viral titers from all viral vectors were quantified at the same time using a SYBR-green real time PCR assay (Bio-Rad) and expressed as viral genomes per milliliter (vg/mL).

**Receptor function measurements**

NRVMs seeded in 6-well plates were transduced with virus containing human and rat RXFP1 virus and Luc control virus at a viral MOI of 10,000 vg/cell. The virus was allowed to transduce the cell for 48 hours before the medium was changed. After 5 days the transduced NRVMs were stimulated with 100 nM recombinant RLN for 5 hours before being harvested for immunoblotting using 1% SDS buffer (1%SDS, 1 mM EDTA, 1 mM EGTA supplemented with protease inhibitor and phosphatase inhibitor cocktail 2 and 3). Proteins were analyzed by SDS-polyacrylamide gel electrophoresis and immunoblotting as previously described (Towbin et al., 1979, Proc. Natl. Acad. Sci. 76: 4350-4354). Membranes were probed with antibodies listed in table 3 overnight. Mouse secondary antibody coupled to Alexa Flour 680 (Invitrogen) and rabbit secondary antibody coupled to Dylight™ 800 (Cell Signaling) were used to detect the signals from the immunoblots utilizing Odyssey infrared imager (LI-COR) detection system. Images were processed using Odyssey imaging software. Significant increase in P-PLB(S16) was observed in the NRVM groups transduced with human or rat RXFP1 virus and both treated with recombinant RLN (Fig. 5A and B), with no significant difference between human and rat RXFP1 virus.

***Example 4 Activation of RXFPI by ML290 (human RXFP1 chemical agonist)***

**RXFP1 activation by *ML290 in vitro***

NRVMs seeded in 6-well plates were transduced with virus containing human RXFP1, rat RXFP1 and Luc control virus at the viral MOI of 10,000 vg/cell. The virus was allowed to transduce the cell for 48 hours before the medium was changed. After 5 days the transduced NRVMs were stimulated with 1 μM of ML290 for 30 minutes before being harvested for immunoblotting using 1% SDS buffer (1%SDS, 1 mM EDTA, 1 mM EGTA supplemented with protease inhibitor and phosphatase inhibitor cocktail 2 and 3). Proteins were analyzed by SDS-polyacrylamide gel electrophoresis and immunoblotting as previously described (Towbin et al., 1979, Proc. Natl. Acad. Sci. 76: 4350-4354). Membranes were probed with antibodies listed in table 3 overnight. Mouse secondary antibody coupled to Alexa Flour 680 (Invitrogen) and rabbit secondary antibody coupled to Dylight™ 800 (Cell Signaling) were used to detect the signals from the immunoblots utilizing Odyssey infrared imager (LI-COR) detection system. Images were processed using Odyssey imaging software. Significant increase in P-PLB(S16) was detected in the NRVMs group transduced with human RXFP1 virus and treated with 1 μM ML290 (Fig. 6A and B). Marked increase in P-PLB(S16) was also observed in the NRVMs group transduced with rat RXFP1 virus and treated with 1 μM ML290. No difference in P-PLB(S16) was observed between human RXFP1 virus and treated with 1 μM ML290 and human RXFP1 virus and treated with 100 nM recombinant RLN.

***Example 5: Inotropic effects of huRXFP1 gene therapy with RLN administration rescue heart failure***

**Virus Production**

For all *in vivo* experiments AAV9 serotype vectors were deployed. The recombinant adeno-associated viral vectors AAV9-CMV-MLC260-FLAG-*Rel1*Rattus (designated AAV9-RXFP1) and AAV9-CMV-MLC260-FLAG-*Rel1*human (designated AAV9-huRXFP1) were generated as follows. Both optimized *Rattus Norvegicus* and *Homo sapiens Rel1* cDNA [NM_201417.1 and NM_021634.3, National Center for Biotechnology Information (NCBI)] were synthesized and cloned into a pCDNA3.1 plasmid with N-terminal FLAG-TAG.After extensive *in vitro* testing, the transgene cassette was subcloned into an AAV transfer plasmid downstream of a cardiac myocyte-specific cytomegalovirus (CMV) enhancer coupled with a short 260bp myosin light chain promoter (MLC260) and the whole construct was flanked by two ITRs. Recombinant vectors were generated by packaging of AAV-inverted terminal repeat recombinant genomes into AAV9 capsids using the two plasmids transfection protocol (Grimm et al., 2003, Mol. Ther. 7: 839-850). High titer vectors were produced in cell stacks (Corning) with polyethylenimine transfection. After 48 hours the vectors were harvested and purified by density filtration in an iodixanol gradient (Jungmann et al., 2017, Hum. Gene. Ther. Methods 28: 235-246). Using the same method, the control vector AAV9-CMV-MLC260-FLAG-*Luc*Firefly (designated AAV9-LUC) was generated. Viral titers from all viral vectors were quantified at the same time using a SYBR-green real time PCR assay (Bio-Rad) and expressed as viral genomes per milliliter (vg/mL).

**Heart Failure Model Generation and Experimental set up**

Heart failure model was generated by transverse aortic constriction operation (TAC) previously described (Rockman et al., 1991, Proc. Natl. Acad. Sci. 88: 8277-8281). 8 weeks old C57BL/6 mice were ordered and weighted prior to the operation. TAC operation was performed on day 0 using 26-gauge blunt needle effectively reducing the diameter of the transverse aorta to approximately 0.46 mm (Rockman et al., 1994, Proc. Natl. Acad. Sci. 91(7): 2694-2698). Cardiac function measurements were performed before Transverse aortic Constriction (TAC) operation (day -2) before virus infusion (day 7), before pump implantation (day 28), and at 2-month follow up time point (day 55) (Fig. 7A). At day 7, animals were randomized to systemic injection of Luciferase control virus or RXFP1 virus. Osmotic pumps were implanted on day 28 with animals randomized to get either saline or recombinant relaxin pumps. Follow up assessments were performed on day 55 and the animals were sacrificed 3 days later. In addition, 17 mice served as control animals that were sham operated followed by either saline or recombinant relaxin pumps implantation.

**Cardiac function measurements**

Echocardiography was performed using Vevo 2100, VisualSonics. The mice were shaved on the chest and held in prone position. Warm ultrasound coupling gel (37° C) was placed on the shaved chest area, and the MS400 transducer was positioned to obtain 2D B-mode parasternal long and short axis views and 1D M-mode short axis view. The ejection fraction (EF) was calculated from 6 consecutive heartbeats in the M-mode using LV trace function.

The EF of all TAC animals were gradually reduced starting from day 7 post operation (EF: $61.49 \pm 8.48\%$). Further reduction was detected in all TAC operated animals on day 28 post operation before the pump implantation (EF: $61.16 \pm 13.47$). The follow up assessments at day 55 showed further reduction in EF in control groups treated with AAV9.LUC receiving either NaCl or RLN (LUC NaCl EF: $51.54 \pm 14.17$ and LUC RLN EF: $50.02 \pm 18.31$). Sight increase in EF was observed in RXFP1 groups treated with NaCl (EF: $60.25 \pm 16.57$) and significant increase in EF was detected in RXFP1 groups treated with RLN (EF: $64.97 \pm 12.78$) (Fig. 7B). Other parameters such as left ventricular internal diameter (LVIdD), Left ventricular mass corrected, and heart rate were also measured, but no significant difference was observed.

**Molecular Analysis**

**RNA expression**

RNA was extracted using TRIzol® reagent (Ambion) from snap frozen tissue (10mg) or cell lysate ($2\times10^6$ cells) according to the manufacturer's protocols. 1 $\mu$g of total the RNA was reverse transcribed into cDNA using the iScript cDNA-Synthesis Kit (Bio-Rad). iQ SYBR Green Supermix was used according to the manufacturer's protocol. A final volume of 15 $\mu$L per reaction consisted of 7.5 $\mu$L iQ SYBR Green Supermix, 1 $\mu$L of forward and reverse primers mix (final concentration of 300 nM each), and 6.5 $\mu$L diluted cDNA prepared (final concentration of 3.25 ng per reaction). The quantitative real-time PCR was performed in duplicate on Bio-Rad CFX96 real-time PCR detection system (Bio-Rad) The $2^{-\Delta\Delta CT}$ method was used to quantify relative gene expression levels between samples. Specificity of PCR products were confirmed by gel electrophoresis. Primers listed in Table 1 above were used for mRNA expression quantification and general RT-PCR program in Table 5 was used for all primers with only adjustment in annealing temperatures being made.

**Table 5:** RT-PCR program

| Step | Temperature | Time | Number of Cycles |
|---|---|---|---|
| Denaturation | 95° C | 3:00 | 1x |
| Denaturation | 95° C | 0:10 | 40x |
| Annealing | Vary annealing Temperature | 0:10 | |
| Elongation and reading | 72° C | 0:30 | |
| Termination | 95° C | 0:10 | 1x |
| Melting Curve | 65 → 95° C with 0.5° C increment | 0:05 | 60x |

Post-mortem mRNA expression analysis using RTPCR revealed a significant increase in RXFP1 mRNA expression within the ventricle of TAC animals treated with huRXFP1 virus (Fig. 7C). Compared to a TAC group treated with control virus alone, a significant decrease in BNP mRNA expression was observed in huRXFPI and RLN treated mice (Fig. 7D).

Other pathogenic cardiac remodeling genes expression was quantified. A trend toward decreasing cardiac remodeling genes *ANP* and *β-MHC* expression was detected in a TAC group that received both huRXFP1 gene therapy and RLN. Furthermore, a trend toward a decrease in collagen production proteins (*Col1a1*, *Col3a1* and *Postn*) was also detected in both huRXFP1 gene therapy with and without RLN treated groups.

### *Example 6: Protective effects of huRXFP1 when highly expressed*

**Transgenic animal Generation**

FLAG huRXFP1 was cloned onto a place holder plasmid containing a full length $\alpha$-MHC promoter flanked by a restriction site. After the correct clone was identified, the plasmid was propagated and the construct including the gene of interest and the promoter were excised from the plasmid using a restriction digest. The correct DNA fragment was identified and purified before given to the Animal facility at the University of Heidelberg for nuclear injection. The transgenic (TG) animal generation protocol was roughly based on Davis et al. (Davis et al., 2012, Circ. Res. 111(6): 761-777).

**Heart Failure Model Generation and Experimental set up**

Heart failure model was generated by transverse aortic constriction operation (TAC) previously described (Rockman et al., 1991, Proc. Natl. Acad. Sci. 88: 8277-8281). 8 weeks old C57BL/6 mice were ordered and weighted prior to the operation. TAC operation was performed on day 0 using a 27-gauge blunt needle effectively reducing the diameter of the transverse aorta to approximately 0.44 mm (Rockman et al., 1994, Proc. Natl. Acad. Sci. 91(7): 2694-2698). Cardiac function measurements were performed before Transverse Aortic Constriction (TAC) operation (day -2) 7 days after the operation (day 7), 28 days after the operation (day 28), and 42 days after the operation (day 42) (Fig. 8A). The animals were sacrificed 2 days after the last assessment. In addition, 20 mice served as control animals that were sham operated.

**Cardiac function measurements**

Echocardiography was performed using Vevo 2100, VisualSonics. The mice were shaved on the chest and held in prone position. Warm ultrasound coupling gel (37° C) was placed on the shaved chest area, and the MS400 transducer was positioned to obtain 2D B-mode parasternal long and short axis views and 1D M-mode short axis view. The ejection fraction (EF) was calculated from 6 consecutive heartbeats in the M-mode using LV trace function.

The EF of all TAC animals were gradually reduced starting from day 7 post operation (EF: 62.80 $\pm$ 12.31%). A further and significant reduction in cardiac function was detected in WT TAC operated animals on day 28 (EF: 58.59 $\pm$ 11.70) compared to TG TAC operated animals (EF: 66.53 $\pm$ 7.33). The follow up assessments at day 42 showed even further reduction in EF in WT TAC animals (EF: 53.79 $\pm$ 14.97) compared to TG TAC animals (EF: 67.96 $\pm$ 8.50) (Fig. 8B). Other parameters such as left ventricular internal diameter (LVIdD), Left ventricular mass corrected, and heart rate were also measured, but no significant different was observed.

**Molecular Analysis**

All RNA was analyzed using primers listed in table 1 above. Post-mortem mRNA expression analysis using RTPCR revealed a significant increase in huRXFP1 mRNA expression within the ventricle of all huRXFP1 transgenic animals (Fig. 8C). Compared to a WT TAC group, a significant decrease in ANP and BNP mRNA expression was observed in TAC operated huRXFPI transgenic animals (Fig. 8C and D).

Other pathogenic cardiac remodeling genes expression was quantified. Furthermore, a significant decrease in collagen production proteins (*Col1a1* and *Col3a1*) was detected in TAC operated huRXFP1 TG animals (Fig. 8E and F).

### *Example 7: Positive inotropic effects of RXFP1 improves intracellular calcium handling in neonatal rat ventricular cardiomyocytes*

**Isolation of Neonatal Rat Ventricular Cardiomyocytes (NRVCM)**

Primary cultures of neonatal rat ventricular cardiac myocytes (NRVCM) were prepared from 1-2 days old Wistar rats (Charles River). The neonatal rats were euthanatized by decapitation and the heart removed and placed in 1X cold ADS solution. The atria and other vessels were removed, and the hearts were transferred into a new 1X cold ADS solution. The ADS solution was removed, and the ventricles were washed with 3 mL of cold ADS before being minced. Tissue fragments were digested in a T75 bottle with 30 mL of a digestion solution containing collagenase and pancreatin for 5 minutes at 37° C. The tissue fragments were allowed to settle on one side of the bottle and the solution removed and discarded. Fresh digestion solution was added, and the tissue fragments were digested for 20 minutes at 37° C. After 20 minutes, the tissue fragments were pipetted up and down 5 times at maximum power and the solution removed and filtered through a 40 $\mu$m cell strainer into a 50 mL falcon. 10 mL of FCS was added through the cell strainer and the solution was centrifuged at 1000 rpm for 5 minutes at room temperature. After centrifugation, the supernatant was removed, and 7 mL of FCS was added. The cell solution was placed in a 37° C incubator awaiting further processing. The tissue fragments were digested at least 5 times in order to completely digest all the tissue pieces. After the digestion, all cell suspensions were combined and centrifuged at 1000 rpm for 5 minutes at room temperature. The supernatant was removed, and the cells were resuspended in 12 mL of cold 1X ADS. The fibroblasts and cardiac myocytes were separated using percoll density gradient. To obtain cardiomyocytes with high purity, two layers Percoll density gradient was performed. The gradient consisted of 63% red Percoll solution, and a 40.5% clear Percoll solution. 4 mL of red Percoll solution was added into each 15 mL falcon. Then 3 mL of clear percoll solution was added using underlaying technique. 2 mL of cell solution containing 1X cold ADS was layered at a 45 degrees angle onto of the gradient. The falcons were centrifuged at 2400 rpm for 30 minutes at 37° C deceleration speed of 0. The upper stromal cells band including fibroblasts was removed by aspiration. The lower cardiomyocytes band was kept and washed twice with cold 1X ADS before being resuspended in a warm Medium 199 supplemented with 10% v/v fetal calf serum, 100 U/mL penicillin, 100 $\mu$g/ streptomycin, 2 mM L-glutamine, and 1 mM calcium chloride. The cells were counted manually in a Neubauer chamber and plated accordingly (Table 6). The NRVCM were cultured at 37° C and 5% $CO_2$ humidified atmosphere. After 48 hours fetal calf serum was reduced to 0.5% and cells were cultured for 2-5 days. Two days after the cells were plated, the medium was removed, and the cells were washed once with warm PBS before starving medium M199 supplemented with 0.5% FCS, 1% penicillin/streptomycin, 1% L-glutamine, 1 mM $CaCl_2$ was added. In the following, the medium was changed every two days until the cells were harvested. Cells were used for the maximum of 7 days before being discarded.

Table 6: Optimal plating density for NRVCMs calcium transient

**Table 6:** Optimal plating density for NRVCMs calcium transient

|  | Area per well (cm$^2$) | Number of cells seeded | Volume per well (mL) |
|---|---|---|---|
| Glass-bottom dish | 24 | $2 \times 10^4$ | 3 |

## Ectopic RXFP1 Expression

NRVCMs seeded in glass-bottom dishes were transduced with RXFP1 virus and LUC control virus with the viral MOI of 10,000 vg/cell. The virus was allowed to transduce the cells for 48 hours before the medium was changed.

## Ca$^{2+}$ Transient Measurement in NRVCMS

Intracellular Ca$^{2+}$ transients were measured according to Yu et al. with modifications to fit available apparatus (Yu et al., 2013, J. Biol. Chem. 288(31): 22481-22492). NRVCMs were transduced with RXFP1 or LUC AAV vectors for 5 days. The medium was changed every two days. On the day of the experiment, the transfected NRVCMs were loaded with 1 $\mu$M Fura 2 AM in M199 medium at 37° C for 15 minutes protected from light. After loading, the cells were washed with warm M199 medium for 15 minutes at 37° C protected from light and connected to an electrode system on the table of an inverted fluorescence microscope (IX70 Olympus). The cells were electrically stimulated with 1 Hz bipolar. Using a monochromator, the Fura 2 AM loaded cells were excited at 380 nm. The alternative measurement of the bispectral emission (340 nm/380 nm) was performed with the fluorescence microscope for a period of 5 minutes and the emission ratio was calculated with the software TILL vision. The data were exported to an online program written by Dr. Martin Bush. The following parameters were analyzed: a) the amplitude of the transient ($\Delta$[340 nm/ 380 nm]) and b) the diastolic Ca$^{2+}$ ([340 nm/ 380 nm]).

A significant increase in calcium amplitude was observed in a group of NRVCMs treated with both RXFP1 and RLN compared to LUC treated with both NaCl and RLN (Fig. 9A and B). Additionally, there was no difference in diastolic calcium detected (Fig. 9C). Lastly, addition mRNA quantification using rat RXFP1 primer from table 1 above was performed and significant rat RXFP1 mRNA expression was observed in NRVCMs that received RXFP1 treatment (Fig. 9D).

*Example 8: Positive inotropic effects of RXFP1 improve intracellular calcium handling in adult cardiomyocytes*

**Isolation of Adult Mouse Cardiomyocytes**

Adult mouse cardiac cells were isolated a modified protocol from Harding et al. (Harding et al., 1990, Cardioscience. 1: 49-54). The mouse heart was perfused with digestion buffer in the Langendorff technique, thus enabling dissociation of single cardiac cells. An adult male mouse (30-35 g) was quickly euthanatized by cervical dislocation. Incision areas on the abdomen and thorax were sterilized with 70% ethanol and opened with lateral cuts by scissors. The abdominal Vena Cava was exposed, and heparin was given I.V. Then the heart was exposed and cut out with a large portion of the aorta and quickly placed into a 6 cm Petri dish with 10 mL of room temperature perfusion buffer. The aorta was slid onto the cannula of the Langendorff apparatus and fixed with a double knots surgical suture. Perfusion was started immediately with a drip rate of ~3.5mL/min. Filling of the coronary arteries was checked and evaluated in order to determine, if the heart was correctly positioned on the cannula. In order to remove residual serum traces and free Ca2+, the heart was washed with perfusion buffer for 1 minute. After that the heart was perfused with digestion buffer containing a mixture of liberase (mixture of collagenases) and trypsin solution for 10 minutes. The perfusion was stopped, when the heart became flaccid and easily penetrable by fine tip forceps. The heart was quickly removed from the cannula and the atria were cut away using surgical scissor. The heart was minced into 1 mm pieces and 2.5 mL stop solution 1 containing 1% BSA and 50 $\mu$M $CaCl_2$ was added to the solution to stop the digestion process. The heart was pipetted up and down using a cut 1 mL pipette tip for 3 minutes. The solution was filtered into a 50 mL falcon and left to precipitate for 15 minutes at room temperature. After the pellet formed, all but 1.5 mL of supernatant was removed and 5 mL of stop solution 2 containing 0.5% BSA and 38 $\mu$M $CaCl_2$ was added to the cells. Calcium treatment was performed after every 4 minutes by adjusting calcium concentration within the solution starting from 62 $\mu$M $CaCl_2$ at 4 minutes, 114 $\mu$M $CaCl_2$ at 8 minutes, 191 $\mu$M $CaCl_2$ at 12 minutes, 498 $\mu$M $CaCl_2$ at 16 minutes, 960 $\mu$M $CaCl_2$ at 20 minutes. After the last adjustment the cell was left to precipitate in the incubator for 12 minutes, and the cells were plated onto 4 laminin coated calcium transient glass bottom dishes. The cells were allowed to attach for 1 hour.

**$Ca^{2+}$ Transient Measurement in Adult Mouse Cardiomyocytes**

Intracellular $Ca^{2+}$ transients were measured according to Yu et al. with modifications to fit available apparatus (Yu et al., 2013, J. Biol. Chem. 288(31): 22481-22492). On the day of the experiment, the adult cardiomyocytes were loaded with 1 $\mu$M Fura 2 AM in BDM free medium at 37° C for 20 minutes protected from light. After loading, the cells were washed with warm BDM free medium for 20 minutes at 37° C. Next the dish was protected from light and connected to an electrode system on the table of a high throughput inverted fluorescence microscope (Cytocypher). The cells were electrically stimulated with 1 Hz bipolar. Using a monochromator, the Fura 2 AM loaded cells were excited at 380 nm. The alternative measurement of the bispectral emission (340 nm/380 nm) was performed with the fluorescence microscope for baseline and after 1, 5, and 10 minutes stimulation with NaCl, 100 nM RLN, and 10 nM ISO. The transients were calculated using transient calculated software from the cytocypher. The following parameters were analyzed: a) Peak Height, b) Departure velocity, and c) Return velocity. A trend toward an increase in all parameters analysis was observed in the isolated TG adult cardiomyocytes after 1 minute of RLN treatment (Fig. 10A - C).

*Example 9: RXFP1 expression analysis in human samples*

**Molecular Analysis**

**RNA expression**

RNA was extracted using TRIzol® reagent (Ambion) from snap frozen tissue (10mg) according to the manufacturer's protocols. 1 $\mu$g of total the RNA was reverse transcribed into cDNA using the iScript cDNA-Synthesis Kit (Bio-Rad). iQ SYBR Green Supermix was used according to the manufacturer's protocol. A final volume of 15 $\mu$L per reaction consisted of 7.5 $\mu$L iQ SYBR Green Supermix, 1 $\mu$L of forward and reverse primers mix (final concentration of 300 nM each), and 6.5 $\mu$L diluted cDNA prepared (final concentration of 3.25 ng per reaction). The quantitative real-time PCR was performed in duplicate on Bio-Rad CFX96 real-time PCR detection system (Bio-Rad) The $2^{-\Delta\Delta CT}$ method was used to quantify relative gene expression levels between samples. Specificity of PCR products were confirmed by gel electrophoresis. Listed primers in table 7 were used for mRNA expression quantification and general RT-PCR program in table 8 was used for all primers with only adjustment in annealing temperatures being made.

**Table 7:** Primers for human samples RT-PCR

| Genes | Primers | Template | SEQ ID NO |
|---|---|---|---|
| *Homo sapiens* *BNP* | Forward primer: 5'- CTTTCCTGGGAGGTCGTTCC - 3' | NM_002521.3 | 32 |
| | Reverse primer: 5'- GTTGCGCTGCTCCTGTAAC -3' | | 33 |
| *Homo sapiens* *Hprt1* | Forward primer: 5'- CTCATGGACTAATTATGGACAGGAC -3' | NM_000194.3 | 34 |
| | Reverse primer: 5'- GCAGGTCAGCAAAGAATTTATAGCC -3' | | 35 |
| *Homo sapiens* *Rel1* | Forward primer2: 5'- GGACCTGAAGGAGCTGTCAC -3' | NM_021634.4 | 36 |
| | Reverse primer2: 5'- TAGGCTGAGAGACTTGAGTTTGAC -3' | | 37 |

**Table 8:** RT-PCR program

| Step | Temperature | Time | Number of Cycles |
|---|---|---|---|
| Denaturation | 95° C | 3:00 | 1x |
| Denaturation | 95° C | 0:10 | |
| Annealing | Vary annealing Temperature | 0:10 | 45x |
| Elongation and reading | 72° C | 0:30 | |
| Termination | 95° C | 0:10 | 1x |
| Melting Curve | 65 → 95° C with 0.5° C increment | 0:05 | 60x |

Ventricular samples from 48 explanted hearts were obtained from University of Heidelberg Biobank. 10 Atria biopsy samples were obtained from AG Constanze Schmidt and 7 healthy ventricular samples were purchased from Biozol (1), Biokatz (5), and amsbio (1). mRNA expression analysis from explanted heart and cardiac atria biopsy using RTPCR revealed a significant increase in BNP mRNA expression within the ventricle of explanted heart compared to healthy control (Fig. 11A). RXFP1 mRNA was significantly expressed in the failing atria compared to non-failing ventricle (Fig. 11B). Lastly, there is a trend toward a decrease in RXFP1 mRNA in the failing ventricle compared to the atria (Fig. 11B). Thus, there is a higher expression of RXFP 1 mRNA in the heart.

***Example 10: Hemodynamics changes in huRXFP1 TG animals after RLN administration***

**Transgenic animal Generation**

FLAG huRXFP1 was cloned onto a place holder plasmid containing a full length $\alpha$-MHC promoter flanked by restriction sites. After the correct clone was identified, the plasmid was propagated and the construct including the gene of interest and the promoter were excised from the plasmid using restriction digest. The correct DNA fragment was identified and purified before given to the Animal facility at the University of Heidelberg for nuclear injection. The transgenic (TG) animal generation protocol was based on Davis et al. (Davis et al. (2012), Circ. Res. 111(6): 761-777).

**Cardiac function measurements**

Pressure-volume loop (PV loop) measurement was performed modified from Abraham and Mao (Abraham and Mao (2015), J. Vis. Exp. (103): e52942). The animal was anesthetized according to the body weight with a cocktail of medetomidine, midazolam, and fentanyl intraperitoneally 15 minutes prior to the measurements. After the righting reflex was lost, the mouse was secured to the operating table with surgical tape. Prior to the measurement, the catheter tip was equilibrated in warm saline for 30 minutes. The neck and chest area was cleaned with ethanol and the fur was removed. An incision was made over the right carotid from mandible to sternum. The surrounding tissue was dissected to expose the right carotid, and the vagus nerve, which runs adjacent to the carotid, was cut. Sterile 6.0 silk suture was placed around the distal end (away from the chest) of the carotid artery, tied and secured. An additional suture was placed below the first suture on the carotid artery. The second suture was loosely tightened and clamped the carotid artery proximally to the second suture. After ensuring that the carotid artery was clamped both proximally and distally, a small incision was made into the carotid artery proximally to the first suture. The catheter tip was inserted into the vessel through the incision and the catheter was secured using the second suture. The catheter was gently advanced into the left ventricle through the carotid guided by the PV loop tracing to ensure correct placement. Different filter settings were applied in order to obtain calculable loops. PV loops recording was started and allowed to stabilize for 10 minutes. After 10 minutes of steady state, 100 $\mu$L of 10 $\mu$g RLN was administered intraperitoneally (I.P.). The PV loops were continuously recorded throughout the experiment until 10 minutes after administration of the tested substances.

Heart rate (HR), $\frac{dP}{dt}$ maximum, and $\frac{dP}{dt}$ minimum were calculated according to the manufacturer's recommendation (Transonic). The average of 20 PV loops between inhalation and exhalation was used for the calculation. A significant increase in HR and $\frac{dP}{dt}$ maximum was observed in a group of RXFP1 transgenic animals receiving RLN compared to WT animals receiving RLN.

Literature:

Abraham and Mao (2015), J. Vis. Exp. (103): e52942
Asokan (2012), Mol Ther 20(4):699
Bish et al. (2008), Human Gene Therapy 19:1359
Davis et al. (2012), Circ. Res. 111(6): 761-777
Grimm et al. (2003), Mol. Ther. 7: 839
Grimm et al. (2006), J Virol 80:426
Harding et al. (1990), Cardioscience. 1: 49-54
Hossain et al. (2011), JBC 286(43):37555
Hu et al. (2016), Biochemistry 55(12):1772
Jungmann et al. (2017), Hum. Gene. Ther. Methods 28: 235
Naso et al. (2017), BioDrugs 31:317
Rockman et al. (1991), Proc. Natl. Acad. Sci. 88: 8277
Rockman et al. (1994), Proc. Natl. Acad. Sci. 91(7): 2694
Smith-Arica and Bartlett (2001), Curr Cardiol Rep 3(1): 43
Teerlink et al. (2013), Lancet 381:29
Towbin et al. (1979), Proc. Natl. Acad. Sci. 76: 4350-4354
Yu et al. (2013), J. Biol. Chem. 288(31): 22481-22492

**Claims**

1. A polynucleotide comprising an expressible nucleic acid sequence encoding a relaxin family peptide receptor 1 (RXFP1) polypeptide for use in treatment and/or prevention of heart failure in a subject, wherein said use further comprises administration of an RXFP1 agonist.

2. The polynucleotide for use of claim 1, wherein said heart failure is chronic heart failure

3. The polynucleotide for use of claim 1 or 2, wherein said polynucleotide comprises the sequence of SEQ ID NO: 1, 3, or 5.

4. The polynucleotide for use of any one of claims 1 to 3, wherein said RXFP1 polypeptide comprises at least the amino acids corresponding to amino acids 1 to 766 of human RXFP 1.

5. The polynucleotide for use of any one of claims 1 to 4, wherein said RXFP1 is human RXFP1, more preferably comprising the amino acid sequence of SEQ ID NO:2 or/and 4 or a sequence at least 70% identical thereto, preferably comprising the amino acid sequence of SEQ ID NO:2 and/or 4.

6. The polynucleotide for use of any one of claims 1 to 5, wherein said polynucleotide comprises, preferably consists of, the sequence of SEQ ID NO:3.

7. The polynucleotide for use of any one of claims 1 to 6, wherein said polynucleotide is comprised in a vector, preferably a viral vector, more preferably an adeno-associated virus (AAV) vector, still more preferably an AAV1, AAV2, AAV5, AAV6, or an AAV9 vector, most preferably an AAV9 vector.

8. The polynucleotide for use of any one of claims 1 to 7, wherein said use comprises intravenous and/or intracardial administration of said polynucleotide to said subject.

9. The polynucleotide for use of any one of claims 1 to 8, wherein said RXFP1 agonist is relaxin or ML290.

10. The polynucleotide for use of any one of claims 1 to 9, wherein said RXFP1 agonist is relaxin.

11. A vector comprising the polynucleotide as specified in any one of claims 1 to 10 for use in treatment and/or prevention of heart failure, wherein said use further comprises administration of an RXFP1 agonist.

12. A host cell comprising the polynucleotide as specified in any one of claims 1 to 10, an RXFP1 polypeptide expressed from a polynucleotide as specified in any one of claims 1 to 10, and/or the vector as specified in claim 11 for use in treatment and/or prevention of heart failure, wherein said use further comprises administration of an RXFP1 agonist.

13. An RXFP1 agonist for use in the treatment of heart failure in a subject, wherein said treatment comprises administration of a polynucleotide as specified in any one of claims 1 to 10, a vector as specified in claim 11, and/or a host cell as specified in claim 12, to said subject.


**Patentansprüche**

1. Polynukleotid, das eine exprimierbare Nukleinsäuresequenz umfasst, die für ein Relaxin-Familie-Peptidrezeptor-1-(RXFP1-)Polypeptid codiert, zur Verwendung bei der Behandlung und/oder Prävention von Herzversagen bei einem Individuum, wobei die Verwendung weiterhin die Verabreichung eines RXFP1-Agonisten umfasst.

2. Polynukleotid zur Verwendung nach Anspruch 1, wobei es sich beim Herzversagen um chronisches Herzversagen handelt.

3. Polynukleotid zur Verwendung nach Anspruch 1 oder 2, wobei das Polynukleotid die Sequenz SEQ ID NO: 1, 3 oder 5 umfasst.

4. Polynukleotid zur Verwendung nach einem der Ansprüche 1 bis 3, wobei das RXFP1-Polypeptid zumindest die Aminosäuren umfasst, die den Aminosäuren 1 bis 766 von humanem RXFP1 entsprechen.

5. Polynukleotid zur Verwendung nach einem der Ansprüche 1 bis 4, wobei es sich beim RXFP1 um humanes RXFP1 handelt, das stärker bevorzugt die Aminosäuresequenz SEQ ID NO: 2 oder/und 4 oder eine zu mindestens 70 ö damit identische Sequenz umfasst, das vorzugsweise die Aminosäuresequenz SEQ ID NO: 2 und/oder 4 umfasst.

6. Polynukleotid zur Verwendung nach einem der Ansprüche 1 bis 5, wobei das Polynukleotid die Sequenz SEQ ID NO: 3 umfasst, vorzugsweise daraus besteht.

7. Polynukleotid zur Verwendung nach einem der Ansprüche 1 bis 6, wobei das Polynukleotid in einem Vektor, vorzugsweise einem viralen Vektor, stärker bevorzugt einem Adeno-assoziierten Virus- (AAV-)Vektor, noch stärker bevorzugt einem AAV1-, AAV2-, AAV5-, AAV6- oder einem AAV9-Vektor, am meisten bevorzugt einem AAV9-Vektor, enthalten ist.

8. Polynukleotid zur Verwendung nach einem der Ansprüche 1 bis 7, wobei die Verwendung eine intravenöse und/oder intrakardiale Verabreichung des Polynukleotids an das Individuum umfasst.

9. Polynukleotid zur Verwendung nach einem der Ansprüche 1 bis 8, wobei es sich beim RXFP1-Agonisten um Relaxin oder ML290 handelt.

10. Polynukleotid zur Verwendung nach einem der Ansprüche 1 bis 9, wobei es sich beim RXFP1-Agonisten um Relaxin handelt.

11. Vektor, der das Polynukleotid gemäß der Beschreibung in einem der Ansprüche 1 bis 10 umfasst, zur Verwendung bei der Behandlung und/oder Prävention von Herzversagen, wobei die Verwendung weiterhin die Verabreichung eines RXFP1-Agonisten umfasst.

12. Wirtszelle, die das Polynukleotid gemäß der Beschreibung in einem der Ansprüche 1 bis 10, ein von einem Polynukleotid gemäß der Beschreibung in einem der Ansprüche 1 bis 10 exprimiertes RXFP1-Polypeptid und/oder den Vektor gemäß der Beschreibung in Anspruch 11 umfasst, zur Verwendung bei der Behandlung und/oder Prävention von Herzversagen, wobei die Verwendung weiterhin die Verabreichung eines RXFP1-Agonisten umfasst.

13. RXFP1-Agonist zur Verwendung bei der Behandlung von Herzversagen bei einem Individuum, wobei die Behandlung die Verabreichung eines Polynukleotids gemäß der Beschreibung in einem der Ansprüche 1 bis 10, eines Vektors gemäß der Beschreibung in Anspruch 11 und/oder einer Wirtszelle gemäß der Beschreibung in Anspruch 12 an das Individuum umfasst.

**Revendications**

1. Polynucléotide comprenant une séquence d'acides nucléiques exprimable codant pour un polypeptide de récepteur peptidique de la famille de la relaxine 1 (RXFP1) pour une utilisation dans le traitement et/ou la prévention d'une défaillance cardiaque chez un sujet, ladite utilisation comprenant en outre une administration d'un agoniste de RXFP1.

2. Polynucléotide pour une utilisation selon la revendication 1, ladite défaillance cardiaque étant une défaillance cardiaque chronique.

3. Polynucléotide pour une utilisation selon la revendication 1 ou 2, ledit polynucléotide comprenant la séquence de la SEQ ID NO: 1, 3 ou 5.

4. Polynucléotide pour une utilisation selon l'une quelconque des revendications 1 à 3, ledit polypeptide RXFP1 comprenant au moins les acides aminés correspondant aux acides aminés 1 à 766 de RXFP1 humain.

5. Polynucléotide pour une utilisation selon l'une quelconque des revendications 1 à 4, ledit RXFP1 étant RXFP1 humain, plus préférablement comprenant la séquence d'acides aminés de la SEQ ID NO: 2 et/ou 4 ou une séquence au moins 70 % identique à celle-ci, préférablement comprenant la séquence d'acides aminés de la SEQ ID NO: 2 et/ou 4.

6. Polynucléotide pour une utilisation selon l'une quelconque des revendications 1 à 5, ledit polynucléotide comprenant, préférablement étant constitué par, la séquence de la SEQ ID NO: 3.

7. Polynucléotide pour une utilisation selon l'une quelconque des revendications 1 à 6, ledit polynucléotide étant compris dans un vecteur, préférablement un vecteur viral, plus préférablement un vecteur de virus adéno-associé (AAV), encore plus préférablement un vecteur AAV1, AAV2, AAV5, AAV6, ou AAV9, le plus préférablement un vecteur AAV9.

8. Polynucléotide pour une utilisation selon l'une quelconque des revendications 1 à 7, ladite utilisation comprenant une administration intraveineuse et/ou intracardiaque dudit polynucléotide audit sujet.

9. Polynucléotide pour une utilisation selon l'une quelconque des revendications 1 à 8, ledit agoniste de RXFP1 étant une relaxine ou ML290.

10. Polynucléotide pour une utilisation selon l'une quelconque des revendications 1 à 9, ledit agoniste de RXFP1 étant une relaxine.

11. Vecteur comprenant le polynucléotide tel que spécifié dans l'une quelconque des revendications 1 à 10 pour une utilisation dans le traitement et/ou la prévention d'une défaillance cardiaque, ladite utilisation comprenant en outre une administration d'un agoniste de RXFP1.

12. Cellule hôte comprenant le polynucléotide tel que spécifié dans l'une quelconque des revendications 1 à 10, un polypeptide RXFP1 exprimé à partir d'un polynucléotide tel que spécifié dans l'une quelconque des revendications 1 à 10, et/ou le vecteur tel que spécifié dans la revendication 11 pour une utilisation dans le traitement et/ou la prévention d'une défaillance cardiaque, ladite utilisation comprenant en outre une administration d'un agoniste de RXFP1.

**13.** Agoniste de RXFP1 pour une utilisation dans le traitement d'une défaillance cardiaque chez un sujet, ledit traitement comprenant une administration d'un polynucléotide tel que spécifié dans l'une quelconque des revendications 1 à 10, d'un vecteur tel que spécifié dans la revendication 11, et/ou d'une cellule hôte telle que spécifiée dans la revendication 12, audit sujet.

Fig. 1

Fig. 1 (continued)

Fig. 2

Fig. 3

Fig. 4

E Phospholamban (S16) Phoshorylation

F

Fig. 4 (continued)

Fig. 5

Fig. 6

Fig. 7

Fig. 7 (continued)

Fig. 8

Fig. 8 (continued)

Fig. 9

C

Diastolic Ca²⁺

F340/F380

**RXFP1 expression**

Fig. 9 (continued)

Fig. 10

Fig. 11

A

B

C

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **GREENBERG et al.** *Lancet,* 2016, vol. 387, 1178
- **BISH et al.** *Human Gene Therapy,* 2008, vol. 19, 1359
- **NASO et al.** *BioDrugs,* 2017, vol. 31, 317
- **ASOKAN.** *Mol Ther,* 2012, vol. 20 (4), 699
- **DEVARAKONDA et al.** *FASEB J*
- **TEERLINK et al.** *Lancet,* 2013, vol. 381, 29
- **HOSSAIN et al.** *JBC,* 2011, vol. 286 (43), 37555
- *J. Mol. Evolution.,* 1987, vol. 25, 351-360
- **HIGGINS et al.** *CABIOS,* 1989, vol. 5, 151-153
- **NEEDLEMAN ; WUNSCH.** *J. Mol. Biol.,* 1970, vol. 48, 443-453
- **SMITH ; WATERMAN.** *Adv. Appl. Math.,* 1981, vol. 2, 482-489
- **GRIMM et al.** *J Virol,* 2006, vol. 80, 426
- **SMITH-ARICA ; BARTLETT.** *Curr Cardiol Rep,* 2001, vol. 3 (1), 43
- **HU et al.** *Biochemistry,* 2016, vol. 55 (12), 1772
- *CHEMICAL ABSTRACTS,* 1482500-76-4
- Remington's Pharmaceutical Sciences. Mack Publishing Company

- **GRIMM et al.** *Mol. Ther.,* 2003, vol. 7, 839-850
- **JUNGMANN et al.** *Hum. Gene. Ther. Methods,* 2017, vol. 28, 235-246
- **ROCKMAN et al.** *Proc. Natl. Acad. Sci.,* 1991, vol. 88, 8277-8281
- **ROCKMAN et al.** *Proc. Natl. Acad. Sci.,* 1994, vol. 91 (7), 2694-2698
- **TOWBIN et al.** *Proc. Natl. Acad. Sci.,* 1979, vol. 76, 4350-4354
- **DAVIS et al.** *Circ. Res.,* 2012, vol. 111 (6), 761-777
- **YU et al.** *J. Biol. Chem.,* 2013, vol. 288 (31), 22481-22492
- **HARDING et al.** *Cardioscience,* 1990, vol. 1, 49-54
- **ABRAHAM ; MAO.** *J. Vis. Exp.,* 2015, vol. 103, e52942
- **ABRAHAM ; MAO.** *J. Vis. Exp.,* 2015, e52942
- **GRIMM et al.** *Mol. Ther.,* 2003, vol. 7, 839
- **JUNGMANN et al.** *Hum. Gene. Ther. Methods,* 2017, vol. 28, 235
- **ROCKMAN et al.** *Proc. Natl. Acad. Sci.,* 1991, vol. 88, 8277
- **ROCKMAN et al.** *Proc. Natl. Acad. Sci.,* 1994, vol. 91 (7), 2694